# EUROPEAN PATENT SPECIFICATION

(11) **EP 1 922 316 B1**
(45) Date of publication and mention of the grant of the patent: **03.03.2010**
(21) Application number: 06790054.8
(22) Date of filing: 28.08.2006
(51) Int. Cl.: C07D 409/14, A61K 31/4184, A61P 35/00

(54) **BENZIMIDAZOLE THIOPHENE COMPOUNDS AS PLK MODULATORS**
BENZIMIDAZOLTHIOPHENVERBINDUNGEN ALS PLK-MODULATOREN
COMPOSÉS DE THIOPHÈNE DE BENZIMIDAZOLE COMME MODULATEURS PLK

(30) Priority: 06.09.2005 US 714303 P
(43) Date of publication of application: 21.05.2008
(73) Proprietor: GlaxoSmithKline LLC, Philadelphia, PA 19102 (US)
(72) Inventor: CHEUNG, Mui, Collegeville, PA 19426 (US); BADIANG, Jennifer Gabriel, Research Triangle Park, NC 27709 (US); DONALDSON, Kelly Horne, Research Triangle Park, NC 27709 (US); RHEAULT, Tara Renae, Reseach Triangle Park, NC 27709 (US)
(74) Representative: Learoyd, Stephanie Anne
(86) International application number: PCT/US2006/033616
(87) International publication number: WO 2007/030359

(56) References cited:
- WO-A-2004/014899

### SEQUENCE LISTING

<110> SmithKline Beecham Corporation
<120> BENZIMIDAZOLE THIOPHENE COMPOUNDS
<130> PR61604
<150> 60/714,303
   <151> 2005-09-06
<160> 2
<170> FastSEQ for Windows Version 4.0
<210> 1
   <211> 11
   <212> PRT
   <213> baculovirus infected T.ni cells
<400> 1
   Met Lys Lys Gly His His His His His His Asp
   1 5 10
<210> 2
   <211> 9
   <212> PRT
   <213> optimized PLK peptide substrate
<400> 2
   Ser Phe Asn Asp Thr Leu Asp Phe Asp
   1 5

## Description

### BACKGROUND OF THE INVENTION

The present invention relates to novel 5-heteroaryl benzimidazole thiophene compounds, pharmaceutical formulations comprising these compounds, and the use of these compounds in therapy.

Polo-like kinases ("PLK") are evolutionarily conserved serine/threonine kinases that play critical roles in regulating processes in the cell cycle. PLK plays a role in the entry into and the exit from mitosis in diverse organisms from yeast to mammalian cells. PLK includes PLK1, PLK2, PLK3 and PLK4.

Overexpression of PLK1 appears to be strongly associated with neoplastic cells (including cancers). A published study has shown high levels of PLK1 RNA expression in >80% of lung and breast tumors, with little to no expression in adjacent normal tissue. Several studies have shown correlations between PLK expression, histological grade, and prognosis in several types of cancer. Significant correlations were found between percentages of PLK-positive cells and histological grade of ovarian and endometrial cancer (p<0.001). These studies noted that PLK is strongly expressed in invading endometrial carcinoma cells and that this could reflect the degree of malignancy and proliferation in endometrial carcinoma. Using RT-PCR analysis, PLK overexpression was detected in 97% of esophageal carcinomas and 73% of gastric carcinomas as compared to the corresponding normal tissues. Further, patients with high levels of PLK overexpression in esophageal carcinoma represented a significantly poorer prognosis group than those with low levels of PLK overexpression. In head and neck cancers, elevated mRNA expression of PLK1 was observed in most tumors; a Kaplan-Meier analysis showed that those patients with moderate levels of PLK1 expression survived longer than those with high levels of PLK1 expression. Analysis of patients with non-small cell lung carcinoma showed similar outcomes related to PLK1 expression.

PCT Publication No. WO2004/014899 to SmithKline Beecham discloses novel benzimidazole thiophene compounds of formula (I): 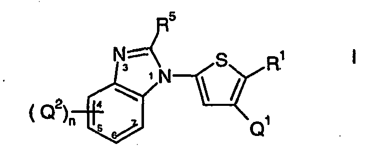 wherein:
R¹ is selected from the group consisting of H, alkyl, alkenyl, alkynyl, -C(O)R⁷, -CO₂R⁷, -C(O)NR⁷R⁸, -C(O)N(R⁷)OR⁸, -C(O)N(R⁷)-R₂-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R⁸, -C(O)N(R⁷)S(O)₂R⁸, -R²-OR⁷, -R²-O-C(O)R⁷, -C(S)R⁷, -C(S)NR⁷R⁸, -C(S)N(R⁷)-Ph, -C(S)N(R⁷)-R²-Ph, -R²-SR⁷, -C(=NR⁷)NR⁷R⁸, -C(=NR⁷)N(R⁸)-Ph, -C(=NR⁷)N(R⁸)-R²-Ph, -R²-NR⁷R⁸, -CN, -OR⁷, -S(O)_{f}R⁷, -S(O)₂NR⁷R⁸, -S(O)₂N(R⁷)-Ph, -S(O)₂N(R⁷)-R²-Ph, -NR⁷R⁸, N(R⁷)-Ph, -N(R⁷)-R²-Ph, -N(R⁷)-SO₂R⁸ and Het;
Ph is phenyl optionally substituted from 1 to 3 times with a substituent selected from the group consisting of halo, alkyl, -OH, -R²-OH, -O-alkyl, -R²-O-alkyl, -NH₂, -N(H)alkyl, -N(alkyl)₂, -CN and -N₃;
Het is a 5-7 membered heterocycle having 1, 2, 3 or 4 heteroatoms selected from N, O and S, or a 5-6 membered heteroaryl having 1, 2, 3 or 4 heteroatoms selected from N, O and S, each optionally substituted from 1 to 2 times with a substituent selected from the group consisting of halo, alkyl, oxo, -OH, -R²-OH, -O-alkyl, -R²-O-alkyl, -NH₂, -N(H)alkyl, -N(alkyl)₂, -CN and -N₃;
Q¹ is a group of formula: -(R²)ₐ-(Y¹)_{b}-(R²)_{c}-R³
a, b and c are the same or different and are each independently 0 or 1 and at least one of a or b is 1;
n is 0, 1, 2, 3 or 4;
Q² is a group of formula: -(R²)ₐₐ-(Y²)_{bb}-(R²)_{cc}-R⁴ or two adjacent Q² groups are selected from the group consisting of alkyl, alkenyl, -OR⁷, -S(O)_{f}R⁷ and -NR⁷R⁸ and together with the carbon atoms to which they are bound, they form a C₅₋₆cycloalkyl, C₅₋₆ cycloalkenyl, phenyl, 5-7 membered heterocycle having 1 or 2 heteroatoms selected from N, O and S, or 5-6 membered heteroaryl having 1 or 2 heteroatoms selected from N, O and S;
aa, bb and cc are the same or different and are each independently 0 or 1; each Y¹ and Y² is the same or different and is independently selected from the group consisting of-O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- and -N(R⁷)C(O)N(R⁷)-;
each R² is the same or different and is independently selected from the group consisting of alkylene, alkenylene and alkynylene;
each R³ and R⁴ is the same or different and is each independently selected from the group consisting of H, halo, alkyl, alkenyl, alkynyl, -C(O)R⁷ , -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii): 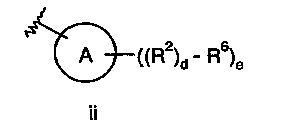 wherein:
   Ring A is selected from the group consisting of C₅₋₁₀cycloalkyl, C₅₋₁₀cycyloalkenyl, aryl, 5-10 membered heterocycle having 1, 2 or 3 heteroatoms selected from N, O and S and 5-10 membered heteroaryl having 1, 2 or 3 heteroatoms selected from N, O and S
   each d is 0 or 1;
   e is 0, 1, 2, 3 or 4;
   each R⁶ is the same or different and is independently selected from the group consisting of H, halo, alkyl, alkenyl, alkynyl, cycloalkyl, cycloalkenyl, Ph, Het, -CH(OH)-R²-OH, -C(O)R⁷ , -CO₂R⁷, -CO₂-R₂-Ph, -CO₂-R²-Het, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C(=NR⁷)NR⁷R⁸, -CR⁷=N-OR⁸, =O -OR⁷, -OC(O)R⁷, -OC(O)Ph, -OC(O)Het, -OC(O)NR⁷R⁸, -O-R²-S(O)₂R⁷, -S(O)_{f}R⁷, -S(O)₂NR⁷R⁸, -S(O)₂Ph, -S(O)₂Het, -NR⁷R⁸, -N(R⁷)C(O)R⁸, -N(R⁷)CO₂R⁸, -N(R⁷)-R₂-CO₂R⁸, -N(R⁷)C(O)NR⁷R⁸, -N(R⁷)-R²-C(O)NR⁷R⁸, -N(R⁷)C(O)Ph, -N(R⁷)C(O)Het, -N(R⁷)Ph, -N(R⁷)Het, -N(R⁷)C(O)NR⁷-R²-NR⁷R⁸; -N(R⁷)C(O)N(R⁷)Ph, -N(R⁷)C(O)N(R⁷)Het, -N(R⁷)C(O)N(R⁷)-R²-Het, -N(R⁷)S(O)₂R⁸, -N(R⁷)-R²-S(O)₂R⁸, -NO₂, -CN and -N₃;
wherein when Q¹ is defined where b is 1 and c is 0, R³ is not halo, -C(O)R⁷ -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN or -N₃;
wherein when Q² is defined where bb is 1 and cc is 0, R⁴ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN or-N₃;
R⁵ is selected from the group consisting of H, halo, alkyl, cycloalkyl, OR⁷, -S(O)_{f}R⁷-NR⁷R⁸, -NHC(O)R⁷, -NHC(O)NR⁷R⁸ and -NHS(O)₂R⁷;
f is 0, 1 or 2; and
each R⁷ and each R⁸ are the same or different and are each independently selected from the group consisting of H, alkyl, alkenyl, alkynyl, cycloalkyl and cycloalkenyl;
wherein when R¹ is -CO2CH₃ and n is 0, Q¹ is not -OH;
or a pharmaceutically acceptable salt, solvate or physiologically functional derivative thereof.

Also disclosed are pharmaceutical compositions containing these compounds, processes for their preparation and methods for treatment of conditions mediated by PLK using these compounds.

### BRIEF SUMMARY OF THE INVENTION

According to a first aspect of the invention there is provided a compound selected from: 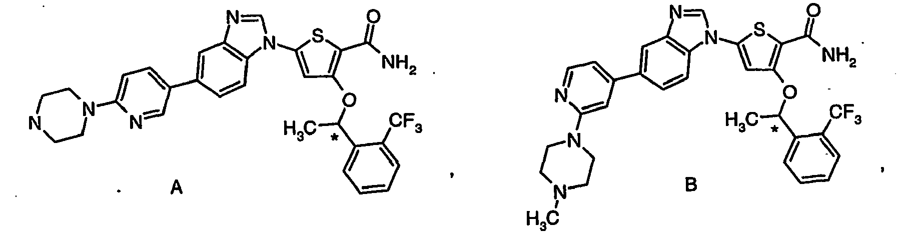 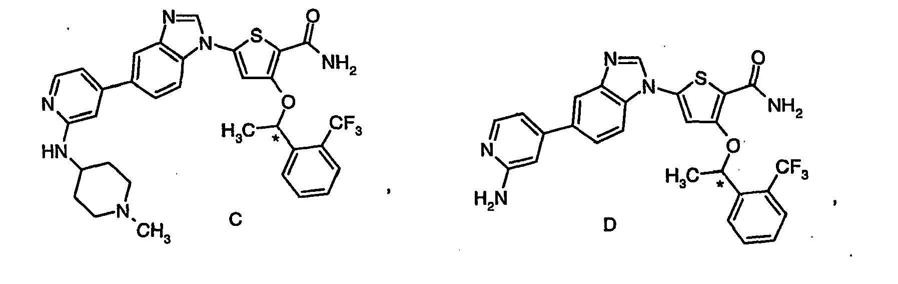 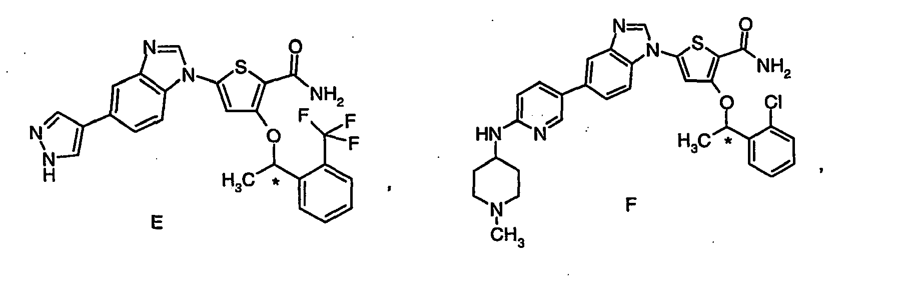 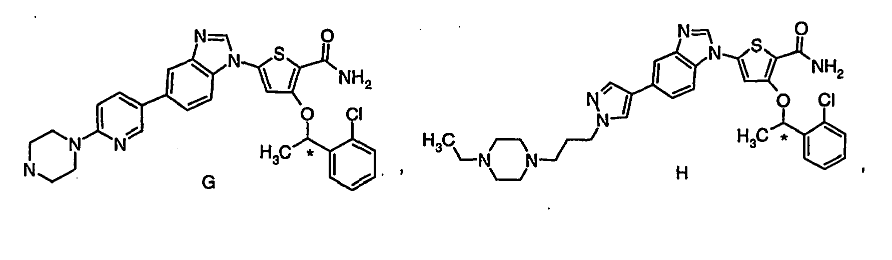 and 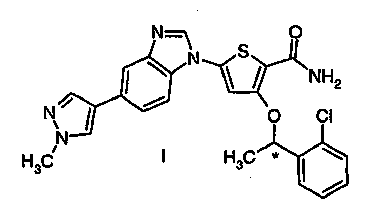 wherein * indicates chiral carbon;
and pharmaceutically acceptable salts or solvates thereof.

In another aspect, there is provided an enantiomerically enriched compound selected from A, B, C, D, E, F, G, H and I wherein the stereochemistry of the chiral carbon is R.

In a third aspect of the invention there is provided a pharmaceutical composition comprising a compound selected from A, B, C, D, E, F, G, H and I and pharmaceutically acceptable salts or solvates thereof. In one embodiment, the pharmaceutical composition further comprises a pharmaceutically acceptable carrier, diluent or excipient.

In another aspect, the present invention provides a compound selected from A, B, C, D, E, F, G, H and I and pharmaceutically acceptable salts or solvates thereof for use in therapy.

In yet another aspect, the present invention provides a compound selected from A, B, C, D, E, F, G, H and I and pharmaceutically acceptable salts or solvates thereof for use in the treatment of a condition mediated by PLK in a mammal in need thereof.

In yet another aspect, the present invention provides a compound selected from A, B, C, D, E, F, G, H and I and pharmaceutically acceptable salts or solvates thereof for use in the treatment of a susceptible neoplasm in a mammal.

In another aspect, the present invention provides a compound selected from A, B, C, D, E, F, G, H and I and pharmaceutically acceptable salts or solvates thereof for use in the treatment of a condition characterized by inappropriate cellular proliferation.

In yet another aspect, the present invention provides a compound selected from A, B, C, D, E, F, G, H and I and pharmaceutically acceptable salts or solvates thereof for use in inhibiting proliferation of a cell.

In yet another aspect, the present invention provides a compound selected from A, B, C, D, E, F, G, H and I and pharmaceutically acceptable salts or solvates thereof for use in inhibiting mitosis in a cell.

In yet another aspect, the present invention provides the use of a compound selected from A, B, C, D, E, F, G, H and I and pharmaceutically acceptable salts or solvates thereof for the preparation of a medicament for the treatment of condition mediated by PLK in a mammal.

In yet another aspect, the present invention provides the use of a compound selected from A, B, C, D, E, F, G, H and I and pharmaceutically acceptable salts or solvates thereof for the preparation of a medicament for the treatment of a susceptible neoplasm in a mammal.

In yet another aspect, the present invention provides the use of a compound selected from A, B, C, D, E, F, G, H and I and pharmaceutically acceptable salts or solvates thereof for the preparation of a medicament for the treatment of a condition characterized by inappropriate cellular proliferation in a mammal.

In yet another aspect, the present invention provides the use of a compound selected from A, B, C, D, E, F, G, H and I and pharmaceutically acceptable salts or solvates thereof for the preparation of a medicament for inhibiting proliferation of a cell.

In yet another aspect, the present invention provides the use of a compound selected from A, B, C, D, E, F, G, H and I and pharmaceutically acceptable salts or solvates thereof for the preparation of a medicament for inhibiting mitosis in a cell.

In yet another aspect, the present invention provides a pharmaceutical composition comprising a compound selected from A, B, C, D, E, F, G, H and I and Pharmaceutical acceptable salts or solvates thereof for use in the treatment of a susceptible neoplasm in a mammal.

### DETAILED DESCRIPTION OF THE INVENTION

As used herein, "compound(s) of the invention" or "compound(s) selected from A, B, C, D, E, F, G, H and I" means compound selected from A, B, C, D, E, F, G, H and I or a pharmaceutically acceptable salt or solvate thereof.

As used herein, "compound(s) selected from A-1, B-1, C-1, D-1, E-1, F-1, G-1, H-1 and I-1" means compound selected from A-1, B-1, C-1, D-1, E-1, F-1, G-1, H-1 and I-1 or a pharmaceutically acceptable salt or solvate thereof.

As used herein, the term "optionally" means that the subsequently described event(s) may or may not occur, and includes both event(s) that occur and events that do not occur.

Compounds of the invention exist in stereoisomeric forms (e.g. they contain one or more chiral or asymmetric carbon atoms). The term "chiral" refers to a molecule that is not superimposable on its mirror image. The term "achiral" refers to a molecule that is superimposable on its mirror image.

The term "stereoisomers" refers to compounds which are have a common chemical constitution but differ in the arrangment of the atoms or groups in space. Stereoisomers may be optical isomers or geometric isomers. Optical isomers include both enantiomers and diastereomers. An "enantiomer" is one of a pair of optical isomers containing a chiral carbon atom whose molecular configuration have left- and right-hand (chiral) forms. That is, "enantiomer" refers to each of a pair of optical isomers of a compound which are non-superimposable mirror images of one another. A "diastereomer" is one of a pair of optical isomers of a compound with two or more centers of dissymmetry and whose molecules are not mirror images of one another. The nomenclature of a chiral center is governed by the (R)-(S) system. Whether a particular compound is designated as the "R" or "S" enantiomer according to the system depends upon the nature of the atoms or groups which are bound to the chiral carbon.

Enantiomers differ in their behavior toward plane-polarized light, that is, their optical activity. An enantiomer that rotates plane-polarized light in a clockwise direction is said to be dextrorotatory and is designated by the symbol "d" our"(+)" for positive rotation. An enantiomer that rotates plane-polarized light in the counterclockwise direction is said to be levorotatory and is designated by the symbol "I" or "(-)" for negative rotation. There is no correlation between the configuration of enantiomers and the direction in which they rotate plane-polarized light. There is also no necessary correlation between the (R) and (S) designation and the direction of rotation of the plane-polarized light. The optical activity, or direction of rotation of plane-polarized light, of an enantiomer of a compound of the invention may be determined using conventional techniques. '

The terms "racemate" and "racemic mixture" as used herein refer to a mixture of the (R)- and the (S)- optical isomers (e.g., enantiomers) of a compound in equal, i.e. 50:50 proportion.

The term "enantiomerically enriched" as used herein refers to preparations comprising a mixture of optical isomers in which the quantity of one enantiomer is higher than the quantity of the other. Thus, "enantiomerically enriched" refers to mixtures of optical isomers wherein the ratio of enantiomer is greater than 50:50. An enantiomerically enriched compound comprises greater than 50% by weight of one enantiomer relative to the other. For example enantiomerically enriched 5-{5-[6-(1-Piperazinyl)-3-pyridinyl]-1*H-*benzimidazol-1-yl}-3-({(1*R*)-1-[2-(trifiuoromethyl)phenyl]ethyl}oxy)-2-thiophenecarboxamide refers to a composition comprising greater than 50% by weight of the (R)-enantiomer relative to the (S)-enantiomer of the compound. In one embodiment, an enantiomerically enriched compound comprises at least 75% by weight of one enantiomer relative to the other. In another embodiment, an enantiomerically enriched compound comprises at least 80% by weight of one enantiomer relative to the other. In one particular embodiment, an enantiomerically enriched compound comprises at least 85% by weight of one enantiomer relative to the other.

The term "enantiomerically pure" as used herein refers to enantiomerically enriched compounds comprising at least 90% by weight of one enantiomer relative to the other. In one embodiment, an enantiomerically pure compound comprises at least 95% by weight of one enantiomer relative to the other. In one particular embodiment, an enantiomerically pure compound comprises at least 99% by weight of one enantiomer relative to the other.

The present invention provides compounds selected from: 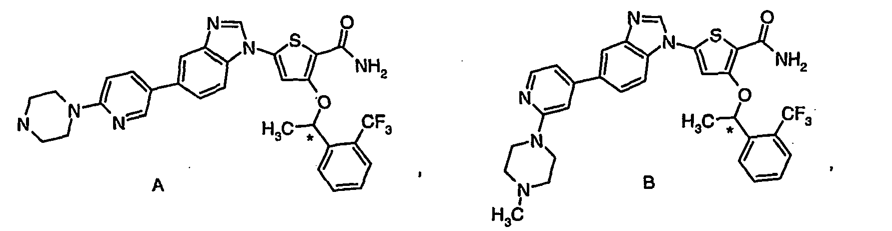 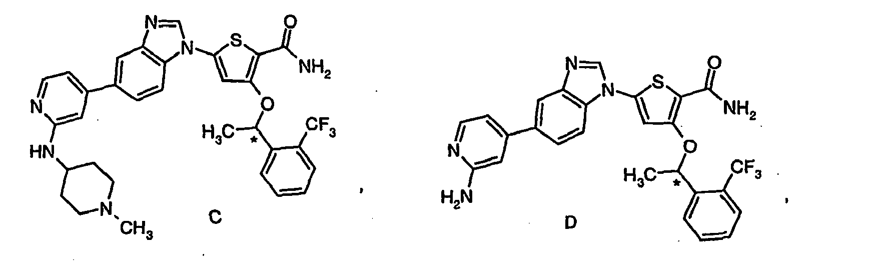 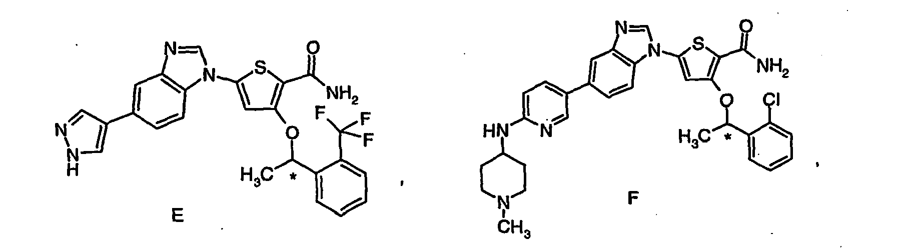 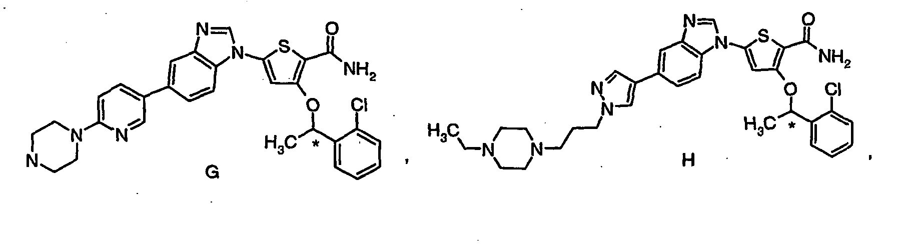 and 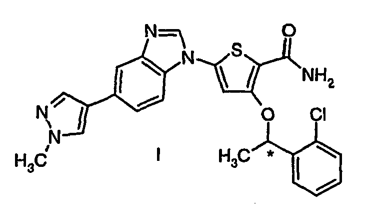 wherein * indicates chiral carbon;
and pharmaceutically acceptable salts and solvates thereof.

In one particular embodiment, the compounds A, B, C, D, E, F, G, H and I are enantiomerically enriched, wherein the stereochemistry of the chiral carbon is R.

Thus, in one preferred embodiment, the present invention provides compounds selected from: 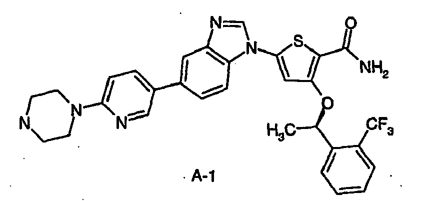 5-{5-[6-(1-Piperazinyl)-3-pyridinyl]-1*H*-benzimidazol-1-yl}-3-({(1*R*)-1-[2-(trifluoromethyl)phenyl]ethyl}oxy)-2-thiophenecarboxamide; 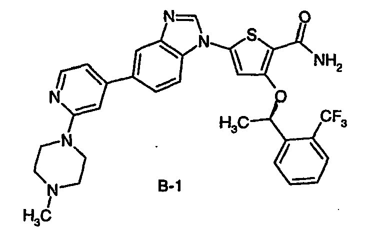 5-{5-[2-(4-Methyl-1-piperazinyl)-4-pyridinyl]-1H-benzimidazol-1-yl}-3-({(1R)-1-[2-(trifluoromethyl)phenyl]ethyl}oxy)-2-thiophenecarboxamide; 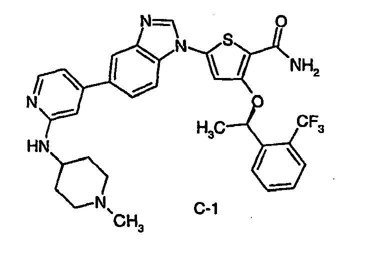 5-(5-{2-[(1-Methyl-4-piperidinyl)amino]-4-pyridinyl}-1H-benzimidazol-1-yl)-3-({(1R)-1-[2-(trifluoromethyl)phenyl]ethyl}oxy)-2-thiophenecarboxamide; 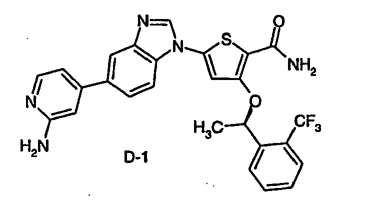 5-[5-(2-Amino-4-pyridinyl)-1H-benzimidazol-1-yl]-3-({(1R)-1-[2-(trifluoromethyl)phenyl]ethyl}oxy)-2-thiophenecarboxamide; 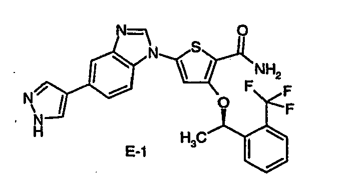 5-[5-(1*H*-Pyrazol-4-yl)-1*H*-benzimidazol-1-yl]-3-({(1*R*)-1-[2-(trifluoromethyl)phenyl]ethyl}oxy)-2-thiophenecarboxamide; 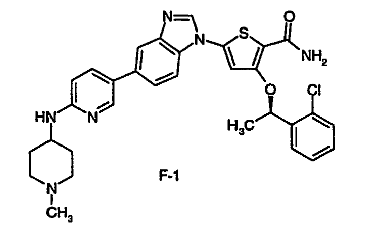 3-{[(1*R*)-1-(2-Chlorophenyl)ethyl]oxy}-5-(5-{6-[(1-methyl-4-piperidinyl)amino]-3-pyridinyl}-1*H*-benzimidazol-1-yl)-2-thiophenecarboxamide; 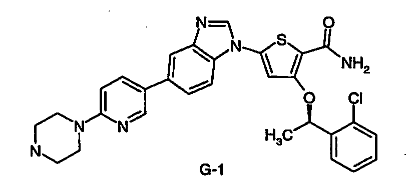 3-{[(1*R*)-1-(2-Chlorophenyl)ethyl]oxy}-5-{5-[6-(1-piperazinyl)-3-pyridinyl]-1*H-*benzimidazol-1-yl}-2-thiophenecarboxamide; 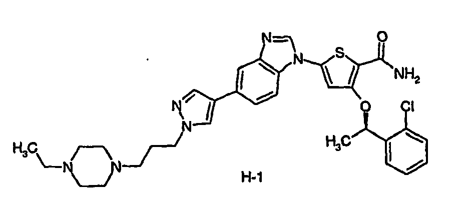 3-{[(1*R*)-1-(2-Chlorophenyl)ethyl]oxy}-5-(5-{1-[3-(4-ethyl-1-piperazinyl)propyl]-1*H*-pyrazol-4-yl}-1*H*-benzimidazol-1-yl)-2-thiophenecarboxamide; 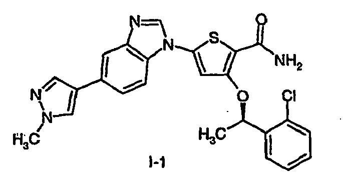 3-{[(1*R*)-1-(2-Chlorophenyl)ethyl]oxy}-5-[5-(1-methyl-1*H*-pyrazol-4-yl)-1*H-*benzimidazol-1-yl]-2-thiophenecarboxamide,
and pharmaceutically acceptable salts and solvates thereof.
It will be appreciated by those skilled in the art that the compounds of the present invention may be utilized in the form of a pharmaceutically acceptable salt or solvate thereof. The pharmaceutically acceptable salts of the compounds of the present invention (or the enantiomerically enriched or pure forms thereof) include conventional salts formed from pharmaceutically acceptable inorganic or organic acids or bases as well as quaternary ammonium salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic (mesylate), naphthalere-2-sulfonic, benzenesulfonic hydroxynaphthoic, hydroiodic, malic, steroic, tannic and the like.

Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts.

The term "solvate" as used herein refers to a complex of variable stoichiometry formed by a solute (a compound of the invention or an enaniomerically enriched or pure form thereof) and a solvent. Solvents, by way of example, include water, methanol, ethanol, or acetic acid.

Processes for preparing pharmaceutically acceptable salts and solvates of the compounds of the invention are conventional in the art. *See,* e.g., Burger's Medicinal Chemistry And Drug Discovery 5th Edition, Vol 1: Principles And Practice.

As will be apparent to those skilled in the art, in the processes described below for the preparation of the compounds of the invention, certain intermediates, may alternatively be in the form of pharmaceutically acceptable salts or solvates of the compound. Those terms as applied to any intermediate employed in the process of preparing the compounds of the invention have the same meanings as noted above with respect to the compounds of the invention. Processes for preparing pharmaceutically acceptable salts and solvates of such intermediates are known in the art and are analogous to the process for preparing pharmaceutically acceptable salts and solvates of the compounds of the invention.

The compounds of the present invention are typically inhibitors of PLK. By PLK inhibitor is meant a compound which exhibits PIC₅₀ greater than 6 in the PLK Inhibition assay described below in the examples or an IC₅₀ less than 10 µM in the Methylene Blue Growth Inhibition assay described below in the examples; more particularly a PLK inhibitor is a compound which exhibits a pIC₅₀ greater than 7 or an IC₅₀ less than 1 µM using the methods described in the examples below.

The present invention further provides compounds of the invention for use in medical therapy in an animal, e.g. a mammal such as a human. In particular, the present invention provides compounds for use in the treatment of a condition mediated by PLK. The present invention also provides compounds for use in the treatment of a susceptible neoplasm. The present invention provides compounds for use in treating a condition characterized by inappropriate cellular proliferation. The present invention also provides compounds for use in inhibiting proliferation of a cell. The present invention also provides compounds for use in inhibiting mitosis in a cell.

As used herein, the term "therapeutically effective amount" means an amount of a compound of the invention which is sufficient, in the subject to which it is administered, to elicit the biological or medical response of a cell culture, tissue, system, animal (including human) that is being sought, for instance, by a researcher or clinician. For example, a therapeutically effective amount of a compound of the invention for the treatment of a condition mediated by PLK is an amount sufficient to treat the PLK mediated condition in the subject. Similarly, a therapeutically effective amount of a compound of the invention for the treatment of a susceptible neoplasm is an amount sufficient to treat the susceptible neoplasm in the subject. In one embodiment of the present invention, the therapeutically effective amount of a compound of the invention is an amount sufficient to inhibit cell mitosis. In one embodiment of the present invention, a therapeutically effective amount of a compound of the invention is an amount sufficient to regulate, modulate, bind or inhibit PLK.

The precise therapeutically effective amount of the compounds of the invention will depend on a number of factors including, but not limited to, the age and weight of the subject being treated, the precise disorder requiring treatment and its severity, the nature of the formulation, and the route of administration, and will ultimately be at the discretion of the attendant physician or vetemarian. Typically, the compound of the invention will be given for treatment in the range of 0.1 to 200 mg/kg body weight of recipient (animal) per day and more usually in the range of 1 to 100 mg/kg body weight per day. Acceptable daily dosages, may be from about 0.1 to about 2000 mg/day, and preferably from about 0.1 to about 100 mg/day.

PLK is essential for cellular mitosis and accordingly, the compounds of the invention are believed to be effective for inhibiting mitosis. "Inhibiting mitosis" refers to inhibiting the entry into the M phase of the cell cycle, inhibiting the normal progression of the M phase of the cell cycle once M phase has been entered and inhibiting the normal exit from the M phase of the cell cycle. Thus, the compounds of the present invention may inhibit mitosis by inhibiting the cell's entry into mitosis, by inhibiting the cell's progression through mitosis or by inhibiting the cell's exit from mitosis.

In one particular embodiment, the cell is a neoplastic cell. In one particular embodiment, the cell is an inappropriately proliferative cell.

The present invention also provides the use of a compound of the invention for the preparation of a medicament for the treatment of condition mediated by PLK in an animal, such as a mammal (e.g., a human). The present invention further provides the use of a compound for the preparation of a medicament for the treatment of a susceptible neoplasm in an animal. The present invention further provides the use of a compound for the preparation of a medicament for the treatment of a condition characterized by inappropriate cellular proliferation. The present invention further provides the use of a compound for the preparation of a medicament for inhibiting proliferation of a cell. The present invention further provides the use of a compound for the preparation of a medicament for inhibiting mitosis in a cell.

While it is possible that, for use in therapy, a therapeutically effective amount of a compound of the invention may be administered as the raw chemical, it is typically presented as the active ingredient of a pharmaceutical composition or formulation. Accordingly, the invention further provides a pharmaceutical composition comprising a compound of the invention. The pharmaceutical composition may further comprise one or more pharmaceutically acceptable carriers, diluents, and/or excipients. The carrier(s), diluent(s) and/or excipient(s) must be acceptable in the sense of being compatible with the other ingredients of the formulation and not deleterious to the recipient thereof. In accordance with another aspect of the invention there is also provided a process for the preparation of a pharmaceutical formulation including admixing a compound of the invention with one or more pharmaceutically acceptable carriers, diluents and/or excipients.

Pharmaceutical formulations may be presented in unit dose form containing a predetermined amount of active ingredient per unit dose. Such a unit may contain a therapeutically effective dose of the compound of the invention or a fraction of a therapeutically effective dose such that multiple unit dosage forms might be administered at a given time to achieve the desired therapeutically effective dose. Preferred unit dosage formulations are those containing a daily dose or sub-dose, as herein above recited, or an appropriate fraction thereof, of an active ingredient. Furthermore, such pharmaceutical formulations may be prepared by any of the methods well known in the pharmacy art.

Pharmaceutical formulations may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions. For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quarternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a seating coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of active ingredient. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax or the like.

The compounds of the invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the invention may also be delivered by the use of monoclonal antibodies as individual carriers to which the compound molecules are coupled. The compounds may also be coupled with soluble polymers as targetable drug carriers. Such polymers can include peptides, polyvinylpyrrolidone, pyran copolymer, polyhydroxypropylmethacrylamide - phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical formulations adapted for topical administration may be formulated as ointments, creams, suspensions, lotions, powders, solutions, pastes, gels, sprays, aerosols or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or as enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient. Pharmaceutical formulations adapted for administration by inhalation include fine particle dusts or mists, which may be generated by means of various types of metered, dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multidose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may include other agents conventional in the art having regard to the type of formulation in question, for example those suitable for oral administration may include flavouring agents.

In the above-described uses, a compound of the invention may be employed alone, in combination with one or more other compounds of the invention or in combination with other therapeutic agents. In particular, in methods of treating conditions mediated by PLK and methods of treating susceptible neoplasms, combination with other chemotherapeutic, hormonal and/or antibody agents is envisaged as well as combination with surgical therapy and radiotherapy. The term "chemotherapeutic" as used herein refers to any chemical agent having a therapeutic effect on the subject to which it is administered. "Chemotherapeutic" agents include but are not limited to anti-neoplastic agents, analgesics and anti-emetics. As used herein, "anti-neoplastic agents" include both cytostatic and cytotoxic agents. Combination therapies thus comprise the administration of at least one compound of the invention and the use of at least one other cancer treatment method. In one particular embodiment, the chemotherapeutic agent is an anti-neoplastic agent. In another embodiment, the present invention provides a pharmaceutical composition as described above further comprising at least one other chemotherapeutic agent, more particularly, the chemotherapeutic agent is an anti-neoplastic agent.

Typically, any chemotherapeutic agent that has activity versus a susceptible neoplasm being treated may be utilized in combination with the compounds of the invention, provided that the particular agent is clinically compatible with therapy employing a compound of the invention. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphor-ines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; and cell cycle signaling inhibitors.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids. Examples of diterpenoids include, but are not limited to, paclitaxel and its analog docetaxel. Examples of vinca alkaloids include, but are not limited to, vinblastine, vincristine, and vinorelbine.

Platinum coordination complexes are non-phase specific anti-neoplastic agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, cisplatin and carboplatin.

Alkylating agents are non-phase anti-neoplastic specific agents and strong electrophiles. Typically, alkylating agents form covalent linkages, by alkylation, to DNA through nucleophilic moieties of the DNA molecule such as phosphate, amino, and hydroxyl groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Antibiotic chemotherapeutic agents are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine and thioguanine.

Camptothecins, including, camptothecin and camptothecin derivatives are available or under development as Topoisomerase I inhibitors.

Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin. Hormones and hormonal analogues are useful compounds for treating cancers in which there is a relationship between the hormone(s) and growth and/or lack of growth of the cancer. Examples of hormones and hormonal analogues believed to be useful in the treatment of neoplasms include, but are not limited to, adrenocorti-costeroids such as prednisone and prednisolone which are useful in the treatment of malignant lymphoma and acute leukemia in children; aminoglutethimide and other aromatase inhibitors such as anastrozole, letrazole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and anti-androgens such as flutamide, nilutamide, bicalutamide, cyproterone acetate and 5α-reductases such as finasteride and dutasteride, useful in the treatment of prostatic carcinoma and benign prostatic hypertrophy; anti-estrogens such as tamoxifen, toremifene, raloxifene, droloxifene and iodoxyfene useful in the treatment of hormone dependent breast carcinoma; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

Signal transduction pathway inhibitors are those inhibitors which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3 domain blockers, serine/threonine kinases, phosphotidyl inositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are sometimes termed growth factor receptors. Inappropriate or uncontrolled activation of many of these kinases, i.e. aberrant kinase growth factor receptor activity, for example by over-expression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr, ErbB2 and ErbB4,), platelet derived growth factor receptor (PDGFr), vascular endothelial growth factor receptor (VEGFR), tyrosine kinase with immunoglobulin-like and epidermal growth factor homology domains (TIE-2), insulin growth factor-I receptor (IGF-I), macrophage colony stimulating factor (cfms), BTK, ckit, cmet, fibroblast growth factor (FGF) receptors, Trk receptors (TrkA, TrkB, and TrkC), ephrin (eph) receptors, and the RET protooncogene. Several inhibitors of growth factor receptors are under development and include ligand antagonists, antibodies, tyrosine kinase inhibitors, anti-sense oligonucleotides and aptamers. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth Factor Receptors as Targets", New Molecular Targets for Cancer Chemotherapy, Ed. Workman, Paul and Kerr, David, CRC Press 1994, London.

Tyrosine kinases, which are not growth factor receptor kinases are termed non-receptor tyrosine kinases. Non-receptor tyrosine kinases useful in the present invention, which are targets or potential targets of anti-neoplastic drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997) Annual Review of Immunology. 15: 371-404.

SH2/SH3 domain blockers are agents that disrupt SH2 or SH3 domains binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (Rafk), Mitogen or Extracellular Regulated Kinase (MEKs), and Extracellular Regulated Kinases (ERKs); and Protein kinase C family member blockers including blockers of subtypes of PKCs (alpha, beta, gamma, epsilon, mu, lambda, iota, zeta), IkB kinase family (IKKa, IKKb), PKB family kinases, Akt kinase family members, and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60.1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; and Martinez-lacaci, L., et al, Int. J. Cancer (2000), 88(1), 44-52.

Inhibitors of Phosphotidyl Inositol-3 Kinase family members including blockers of PI3-kinase, ATM, DNA-PK, and Ku are also useful in combination with the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997), International Journal of Biochemistry and Cell Biology. 29 (7):935-8; and Zhong, H. et al, Cancer Res, (2000) 60(6), 1541-1545.

Also useful in combination with the present invention are Myo-inositol signaling inhibitors such as phospholipase C blockers and Myoinositol analogues. Such signal inhibitors are described in Powis, G., and Kozikowski A., (1994) New Molecular Targets for Cancer Chemotherapy ed., Paul Workman and David Kerr, CRC Press 1994, London.

Another group of signal transduction pathway inhibitors useful in combination with the present invention are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block Ras activation in cells containing wild type mutant Ras, thereby acting as antiproliferation agents. Ras oncogene inhibition is discussed in Scharovsky, O.G., Rozados, V.R., Gervasoni, S.I. Matar, P. (2000), Journal of Biomedical Science. 7(4) 292-8; Ashby, M.N. (1998), Current Opinion in Lipidology. 9(2)99-102; and BioChim. Biophys. Acta, (1989) 1423(3):19-30.

As mentioned above, antibodies to receptor kinase ligand binding may also serve as signal transduction inhibitors. This group of signal transduction pathway inhibitors includes the use of humanized antibodies to the extracellular ligand binding domain of receptor tyrosine kinases. For example, Imclone C225 EGFR specific antibody (see Green, M.C. et al, Monoclonal Antibody Therapy for Solid Tumors, Cancer Treat. Rev., (2000), 26(4), 269-286); Herceptin® ErbB2 antibody (see Tyrosine Kinase Signaling in Breast Cancer:ErbB Family Receptor Tyrosine Kinases, Breast Cancer Res., 2000, 2(3), 176-183); and 2CB VEGFR2 specific antibody (see Brekken, R.A. et al, Selective Inhibition of VEGFR2 Activity by a Monoclonal Anti-VEGF Antibody Blocks Tumor Growth in Mice, Cancer Res. (2000) 60, 5117-5124).

Receptor kinase angiogenesis inhibitors may also find use in the present invention. Inhibitors of angiogenesis related VEGFR and TIE2 are discussed above in regard to signal transduction inhibitors (both receptors are receptor tyrosine kinases). Other inhibitors may be used in combination with the compounds of the present invention. For example, anti-VEGF antibodies, which do not recognize VEGFR (the receptor tyrosine kinase), but bind to the ligand; small molecule inhibitors of integrin (alpha, beta₃) that will inhibit angiogenesis; endostatin and angiostatin (non-RTK) may also prove useful in combination with PLK inhibitors.

Agents used in immunotherapeutic regimens may also be useful in combination with the compounds of the invention.

Agents used in proapoptotic regimens (e.g., bcl-2 antisense oligonucleotides) may also be used in the combination of the present invention. Members of the Bcl-2 family of proteins block apoptosis. Upregulation of bcl-2 has therefore been linked to chemoresistance. Studies have shown that the epidermal growth factor (EGF) stimulates anti-apoptotic members of the bcl-2 family (i.e., mcl-1). Therefore, strategies designed to downregulate the expression of bcl-2 in tumors have demonstrated clinical benefit and are now in Phase II/III trials, namely Genta's G3139 bcl-2 antisense oligonucleotide. Such proapoptotic strategies using the antisense oligonucleotide strategy for bcl-2 are discussed in Water JS et al., J. Clin. Oncol. 18:1812-1823 (2000); and Kitada S et al., Antisense Res. Dev. 4:71-79 (1994).

Cell cycle signaling inhibitors inhibit molecules involved in the control of the cell cycle. Cyclin dependent kinases (CDKs) and their interaction cyclins control progression through the eukaryotic cell cycle. The coordinated activation and inactivation of different cyclin/CDK complexes is necessary for normal progression through the cell cycle. Several inhibitors of cell cycle signaling are under development For instance, examples of cyclin dependent kinases, including CDK2, CDK4, and CDK6 and inhibitors for the same are described in, for instance, Rosania, et al., Exp. Opin. Ther. Patents 10(2):215-230 (2000).

When a compound of the invention is used in combination with a chemotherapeutic agent, the dose of each compound may differ from that when the compound is used alone. Appropriate doses will be readily appreciated by those skilled in the art. The appropriate dose of the compound(s) of the invention and the other therapeutically active agent(s) and the relative timings of administration will be selected in order to achieve the desired combined therapeutic effect, and are within the expertise and discretion of the attendent clinician.

The compounds of the invention may be conveniently prepared by the methods described in the examples which follow.

The following examples are intended for illustration only and are not intended to limit the scope of the invention in any way, the invention being defined by the claims which follow.

The following abbreviations as employed in the examples, have the recited meanings.

| | |
|---|---|
| g | gram(s) |
| mg | milligram(s) |
| mol | mole(s) |
| mmol | millimole(s) |
| N | normal |
| M | Molar |
| mM | millimolar |
| µM | micromolar |
| nM | nanomolar |
| L | liter(s) |
| mL | milliliter(s) |
| µL | microliter(s) |
| h | hour(s) |
| min | minute(s) |
| °C | degrees Centigrade |
| HCl | hydrochloric acid |
| DCM | dichloromethane |
| MeOH | methanol |
| EtOAc | ethyl acetate |
| MgSO₄ | magnesium sulfate |
| NaHCO₃ | sodium bicarbonate |
| K₂CO₃ | potassium carbonate |
| Na₂SO₄ | sodium sulfate |
| N₂ | nitrogen |
| H₂ | hydrogen |

XANTPHOS (4,5-Bis(diphenylphosphino)-9,9-dimethylxanthene) is a catalyst commercially available from Aldrich.

Reagents are commercially available or are prepared according to procedures in the literature. In the following structures, "Me" refers to the group -CH₃.

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Varian VXR-300, a Varian Unity-300, a Varian Unity-400 instrument, or a General Electric QE-300. Chemical shifts are expressed in parts per million (ppm, δ units). Coupling constants are in units of hertz (Hz). Splitting patterns describe apparent multiplicities and are designated as s (singlet), d (doublet), t (triplet), q (quartet), m (multiplet), br (broad).

Low-resolution mass spectra (MS) were recorded on a JOEL JMS-AX505HA, JOEL SX-102, or a SCIEX-APliii spectrometer; high resolution MS were obtained using a JOEL SX-102A spectrometer. All mass spectra were taken under electrospray ionization (ESI), chemical ionization (Cl), electron impact (EI) or by fast atom bombardment (FAB) methods. Infrared (IR) spectra were obtained on a Nicolet 510 FT-IR spectrometer using a 1-mm NaCl cell. All reactions were monitored by thin-layer chromatography on 0.25 mm E. Merck silica gel plates (60F-254), visualized with UV light, 5% ethanolic phosphomolybdic acid or p-anisaldehyde solution or mass spectrometry (electrospray or AP). Flash column chromatography was performed on silica gel (230-400 mesh, Merck) or using automated silica gel chromatography (Isco, Inc. Sq 16x or 100sg Combiflash).

Reported HPLC retention times (RT) were obtained on a Waters 2795 instrument attached to a Waters 996 diode array detector reading 210-500 nm. The column used was a Synergi Max-RP (50 x 2 mm) model #00B-4337-B0. Solvent gradient was 15% MeOH:water to 100% MeOH (0.1 % formic acid) over 6 min. Flow rate was 0.8 mL/min. Injection volume was 3 microliters.

### Intermediate Example 1: Methyl 5-amino-3-({(1R)-1-[2-(trifluoromethyl)-phenyl]ethyl}oxy)-2-thiophenecarboxylate

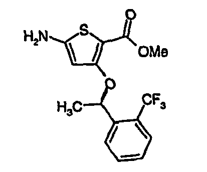

### Step A - Methyl 5-nitro-3-({(1R)-1-[2-(triofluoromethyl)phenyl]ethyl}oxy)-2-thiophenecarboxylate

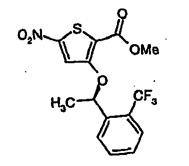

A slurry of polymer-supported triphenylphosphine (62.36 g, 2.21 mmol/g, 137.8 mmol) in DCM (1.0 L) was stirred at room temperature for 10 minutes. The mixture was cooled to 0 °C. Methyl 3-hydroxy-5-nitro-2-thiophenecarboxylate (20.00 g, 98.44 mmol), which may be prepared in a manner analogous to the literature procedure (Barker, J.M.; Huddleston, P.R.; Wood, M.L; Burkitt, S.A. Journal of Chemical Research (Miniprint) 2001, 1001-1022) was added, followed by (1*S*)-1-[2-(trifluoromethyl)phenyl]ethanol (26.20 g, 137.8 mmol) and di-*tert*-butyl azodicarboxylate (31.73 g, 137.8 mmol). The reaction mixture was stirred at room temperature for 21.25 h and then was filtered through a fritted funnel and concentrated. The residue was treated with 4 N HCl in 1,4-dioxane (300 mL) and stirred at room temperature for 3 h. The mixture was then quenched by addition of 3 N sodium hydroxide (300 mL) and saturated aqueous NaHCO₃ (200 mL). The mixture was extracted with DCM (3 x 250 mL). The combined organic fractions were dried over MgSO₄, filtered, and concentrated onto silica gel. Purification by column chromatography (0 to 25% EtOAc:hexanes) provided 36.08 g (98%) of the title compound as yellow oil. ¹H NMR (300 MHz, CDCl₃): δ 7.82 (d, 1H, *J*= 7.8 Hz), 7.68 (d, 1H, *J*= 7.8 Hz), 7.59 (t, 1H, *J*= 7.4 Hz), 7.46 (s, 1H), 7.42 (t, 1H, *J=* 7.6 Hz), 5.77 (q, 1H, *J=* 6.1 Hz), 3.94 (s, 3H), 1.74 (d, 3H, *J*= 6.1 Hz).

### Step B - Methy/5-amino-3-({(1R)-1-[2-(trif/uoromethyl)phenyl]ethyl}oxy)-2-thiophenecarboxylate

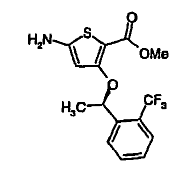

To a flask equipped with a temperature probe, an overhead mechanical stirrer, a reflux condenser, and an addition funnel was added iron powder (26.84 g, 480.6 mmol) and acetic acid (130 mL). The iron/acetic acid slurry was stirred mechanically and heated to an internal temperature of 50 °C. To the addition funnel was added a solution of methyl 5-nitro-3-({(1*R*)-1-[2-(trifluoromethyl)phenyl]ethyl}oxy)-2-thiophenecarboxylate (36.08 g, 96.13 mmol) in acetic acid (160 mL). The solution in the addition funnel was then added dropwise to the iron/acetic acid slurry at a rate such that the internal temperature was maintained at <60 °C (2.5 h total addition time). The reaction mixture was cooled to room temperature, diluted with DCM (500 mL), and then quenched by addition of 6 N sodium hydroxide (750 mL) and saturated aqueous NaHCO₃ (200 mL). The entire mixture was then filtered through a pad of Celite to remove insoluble material, rinsing the Celite with additional DCM (250 mL). The aqueous and organic fractions were separated. The aqueous fraction was extracted with EtOAc (2 x 400 mL). The organic fractions were combined, dried over MgSO₄, filtered, and concentrated to afford 30.66 g (92%) of the title compound as an orange solid. ¹H NMR (300 MHz, CDCl₃): δ 7.89 (d, 1H, *J*= 7.7 Hz), 7.62 (d, 1H, *J*= 7.7 Hz), 7.56 (t, 1H, *J*= 7.7 Hz), 7.36 (t, 1H, *J*= 7.7 Hz), 5.72 (s, 1H). 5.65 (q, 1H, *J*= 6.3 Hz), 4.26 (br s, 2H), 3.80 (s, 3H), 1.66 (d, 3H, *J*= 6.3 Hz); MS (APCI): 368.00 [M+Na]⁺.

### Intermediate Example 2: Methyl 5-amino-3-{[(1R)-1-(2-chlorophenyl)ethyl]-oxy}-2-thiophenecarboxylate

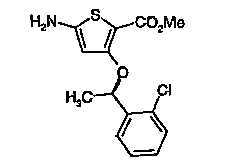

### Step A - Methyl3-{[(1R)-1-(2-chlorophenyl)ethyl]oxy}-5-nitro-2-thiophenecarboxylate

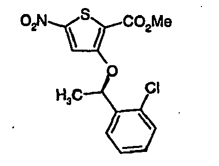

The title compound was prepared from methyl 3-hydroxy-5-nitro-2-thiophenecarboxylate and (1S)-1-(2-chlorophenyl)ethanol by a procedure analogous to Intermediate Example 1, Step A. ¹H NMR (400 MHz, DMSO-d₆): δ 7.96 (s, 1H), 7.65 (dd, 1H, *J* = 1.7, 7.8 Hz), 7.47 (dd, 1H, *J*= 1.5, 7.7 Hz), 7.40 (dt, 1H, *J*=1.3, 7.5 Hz), 7.34 (dt, 1H, *J*=1.9, 7.5 Hz), 5.98 (q, 1H, *J* = 6.0 Hz), 3.85 (s, 3H), 1.59 (d, 3H, *J*= 6.2 Hz).

### Step B - Methyl 5-amino-3-{[(1R)-1-(2-chlorophenyl)ethyl]oxy}-2-thiophenecarboxylate

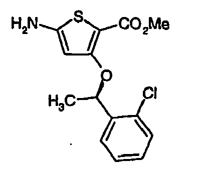

The title compound was prepared from methyl 3-{[(1*R*)-1-(2-chlorophenyl)ethyl]oxy}-5-nitro-2-thiophenecarboxylate by a procedure analogous to Intermediate Example 1, Step B. ¹H NMR (400 MHz, DMSO-*d₆*): δ 7.54 (dd, 1H, *J*= 1.8, 7.9 Hz), 7.45 (dd, 1H, *J*= 1.4, 7.7 Hz), 7.37 (dt, 1H, *J*=1.4, 7.7 Hz), 7.31 (dt, 1H, *J*= 1.8, 7.6 Hz), 6.76 (br s, 2H), 5.57 (q, 1H, *J*= 6.2 Hz), 5.49 (s, 1H), 3.63 (s, 3H), 1.51 (d, 3H, *J*= 6.4 Hz); MS (ESI): 334.03 [M+Na]⁺.

### Example 1: 5-{5-[6-(1-Piperazinyl)-3-pyridinyl]-1H-benzimidazol-1-yl}-3-({(1R)-1-[2-(trifluoromethyl)phenyl]ethyl}oxy)-2-thiophenecarboxamine

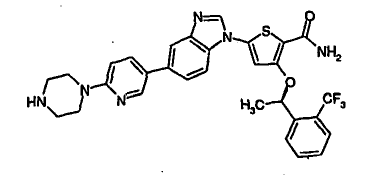

### Step A - Methyl 5-[(4-bromo-2-nitrophenyl)amino]-3({(1R)-1-[2-(trifluoromethyl)phenyl]ethyl}oxy)thiophene-2-carboxylate

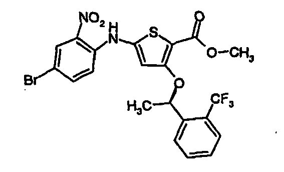

To a stirred mixture of methyl 5-amino-3-({(1*R*)-1-[2-(trifluoromethyl)phenyl]ethyl}oxy)-2-thiophenecarboxylate (10.0 g, 29.0 mmol), 2,5-dibromonitrobenzene (8.13 g, 29.0 mmol), tris(dibenzylideneacetone)dipalladium (0) (0.53 g, 0.58 mmol), and 4,5-bis(diphenylphosphino)-9,9 dimethylxanthene (0.73 g, 1.3 mmol) in toluene (100 mL) under N₂ was added cesium carbonate (47.0 g, 144 mmol). The reaction mixture was stirred at 55 °C for 1.75 h after which time the mixture was cooled slightly and concentrated to dryness *in vacuo.* The solids were slurried in DCM and filtered. The filtrate was concentrated onto silica gel. Purification by column chromatography (3 to 40% EtOAc:hexanes) provided 9.69 g (61%) of the title compound as a red foam. ¹H NMR (400 MHz, CDCl₃): δ 9.46 (s, 1H), 8.31 (d, 1H, *J*= 2.4 Hz), 7.88 (s, 1H, *J*= 8.0 Hz), 7.61 (m, 2H), 7.47 (m, 2H), 7.10 (d, 1H, *J*= 8.8 Hz), 6.42 (s, 1H), 5.70 (m, 1H), 3.86 (s, 3H), 1.71 (d, 3H, *J*= 6.4 Hz).

### Step B - Methyl5-[(2-amino-4-bromophenyl)amino]-3-({(1R)-1-[2-(trifluoromethyl)phenyl]ethyl}oxy)thiophene-2-carboxy/ate

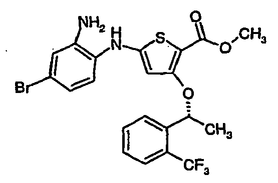

Methyl 5-[(4-bromo-2-nitrophenyl)amino]-3-({(1*R*)-1-[2-(trifluoromethyl)phenyl]-ethyl}oxy)thiophene-2-carboxylate (8.54 g, 15.7 mmol) and sulfided platinum (5 wt% on carbon, 3.05 g, 0.785 mmol) were stirred in EtOAc (250 mL) under 3.79 bar (55 psi) of H₂ for 1.25 h after which time the mixture was filtered and concentrated to give the title compound as a yellow foam (8.66 g crude product). MS (ESI): 516.0 [M]⁺.

### Step C - Methy/5-(5-bromo-1H-benzimidazo/-1-yl)-3-({(1R)-1-[2-(trifluoromethyl)-pheny]ethyl}oxy)thiophene-2-carboxylate

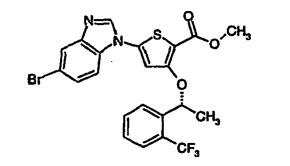

Methyl 5-[(2-amino-4-bromophenyl)amino]-3-({(1*R*)-1-[2-(trifluoromethyl)phenyl]-ethyl}oxy)thiophene-2-carboxylate (8.66 g crude product) and a catalytic amount of pyridinium p-toluenesulfonate (^{~}20 mg) were stirred in triethyl orthoformate:DCM (30 mL:10 mL) at room temperature for 45 min after which time the reaction mixture was purified by column chromatography (0-30-50% EtOAc:hexanes). The resulting foam was triturated in ether to give 6.04 g (73%, 2 steps) of the title compound as a gray solid. ¹H NMR (400 MHz, CDCl₃): δ 7.90 (m, 3H), 7.61 (m, 2H), 7.41 (m, 2H), 7.28 (d, 1H, *J*= 8.4 Hz), 6.72 (s, 1H), 5.78 (m, 1H), 3.90 (s, 3H), 1.75 (d, 3H, *J* = 6.4 Hz).

### Step D - 1,1-Dimethylethyl 4-(5-{1-[5-[(methyloxy)carbonyl]-4-({(1R)-1-[2-(trifluoromethyl)phenyl]ethyl}oxy)-2-thienyl]-1H-benzimidazol-5-yl}-2-pyridinyl)-1-piperazinecarboxy/ate

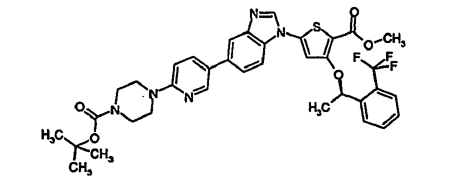

1,1-Dimethylethyl 4-[5-(4,4,5,5-tetramethyl-1,3,2-dioxaborolan-2-yl)-2-pyridinyl]-1-piperazinecarboxylate (1.0 g, 2.5 mmol) was suspended in *N,N-*dimethylacetamide (14 mL) and treated with aqueous 1 N sodium carbonate (6 mL, 6 mmol), methyl 5-(5-bromo-1*H*-benzimidazol-1-yl)-3-({(1*R*)-1-[2-(trifluoromethyl)phenyl]ethyl}oxy)-2-thiophenecarboxylate (800 mg, 1.5 mmol) and 1,1'-bis diphenylphosphinoferrocene dichloropalladium(II) DCM adduct (210 mg, 0.26 mmol) and heated to 80 °C for 1.5 h. The reaction was allowed to cool to room temperature overnight. The reaction mixture was partitioned between 5:1 chloroform:MeOH and saturated aqueous NaHCO₃. Toluene was added and the mixture concentrated (2x) to remove remaining *N*,*N-*dimethylacetamide. The crude compound was purified by column chromatography (hexanes:EtOAc, with 0.5% triethylamine) to yield 1.0 g of the title compound as an oil. MS (APCI): 708.15 [M+H]⁺.

### Step E - 1,1-Dimethylethyl 4-(5-{1-[5-(aminocarbonyl)-4-({(1R)-1-[2-(trifluoromethyl)phenyl]ethyl}oxy)-2-thieny]-1H-benzimidazol-5-yl}-2-pyridinyl)-1 piperazinecarboxylate

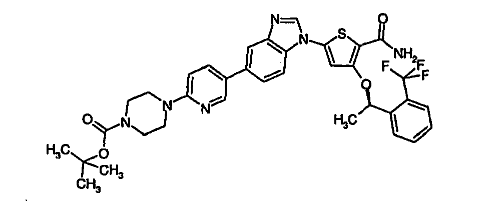

1,1-Dimethylethyl 4-(5-{1-[5-[(methyloxy)carbonyl]-4-({(1*R*)-1-[2-(trifluoromethyl)phenyl]ethyl}oxy)-2-thienyl]-1*H*-benzimidazol-5-yl}-2-pyridinyl)-1-piperazinecarboxylate (1.0 g, 1.41 mmol) was dissolved in 7 N ammonia in MeOH (15 mL) and placed in a sealed vessel. The mixture was heated at 80 °C for 16 h and then cooled to room temperature. The crude mixture was purified by column chromatography (hexanes:EtOAc, with 0.5% triethylamine) to yield 810 mg of the title compound as a yellow foam. MS (APCI): 693.11 [M+H]⁺.

### Step F- 5-{5-[6-(1-Piperazinyl)-3-pyridinyl]-1H-benzimidazol-1-yl}-3-({(1R)-1-[2-(trifluoromethyl)phenyl]ethyl}oxy)-2-thiophenecarboxamide

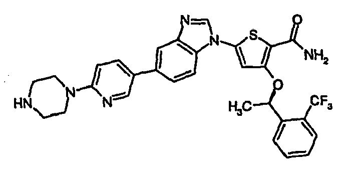

1,1-Dimethylethyl 4-(5-{1-[5-(aminocarbonyl)-4-({(1*R*)-1-[2-(trifluoromethyl)phenyl]ethyl}oxy)-2-thienyl]-1*H*-benzimidazol-5-yl}-2-pyridinyl)-1-piperazinecarboxylate (810 mg, 1.17mmol) in chloroform (20 mL) was treated with trifluoroacetic acid (3 mL) and stirred at room temperature for 1.5 h. The reaction was quenched by the slow addition of saturated aqueous NaHCO₃ until the pH was >7. The reaction mixture was partitioned between 5:1 chloroform:MeOH and saturated aqueous NaHCO₃. The organic layer was dried over Na₂SO₄. The mixture was filtered, concentrated and purified by column chromatography (90/9/1 DCM/MeOH/ammonium hydroxide:DCM). The product was triturated in diethyl ether and filtered to yield 277 mg of the title compound as a light yellow solid. ¹H NMR (400 MHz, DMSO-*d₆*): δ 8.53 (s, 1H), 8.47 (d, 1H, *J*= 2.4 Hz), 7.95-7.87 (m, 3H), 7.82 (br s, 1H), 7.77-7.74 (m, 2H), 7.61-7.50 (m, 3H), 7.12 (br s, 2H), 6.86 (d, 1H, *J*= 8.8 Hz), 5.94 (q, 1H, *J=* 6.0 Hz), 3.42 (t, 4H, *J*= 4.9 Hz), 2.76 (t, 4H, *J=* 4.9 Hz), 1.73 (d, 3H, *J* = 6.0 Hz); HRMS C₃₀H₂₈N₆O₂F₃S: [M+H]⁺ calc'd. 593.1947, found 593.1942.

### Example 2: 5-{5-[2-(4-Methyl-1-piperazinyl)-4-pyridinyl]-1H-benzimidazol-1-yl}-3-({(1R)-1-[2-(trifluoromethyl)phenyl)ethyl}oxy)-2-thiophenecarboxamide

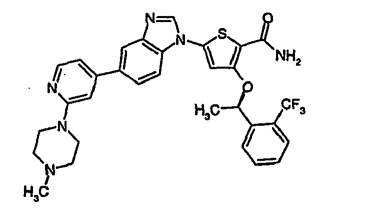

### Step A - 5-(5-Bromo-1H-benzimidazol-1-yl)-3-({(1R)-1-[2-(trifluoromethyl)phenyl]-ethyl}oxy)thiophene-2-carboxamide

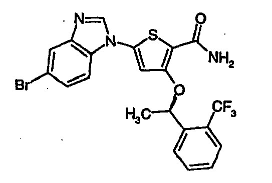

The title compound was prepared from methyl 5-(5-bromo-1*H*-benzimidazol-1-yl)-3-({(1*R*)-1-[2-(trifluoromethyl)phenyl]ethyl}oxy)thiophene-2-carboxylate (3.98 g, 7.58 mmol) and 100 mL 7 N ammonia in MeOH heating in a sealed vessel at 95 °C for 72 h. The mixture was cooled to room temperature and filtered to give a yellow solid. The remaining filtrate was purified by column chromatography (0 to 20% MeOH:DCM), and the resulting solid was combined with the above yellow solid to give 3.21 g (83%) of the title compound. ¹H NMR (400 MHz, CDCl₃): δ 7.94 (d, 1H, *J*= 1.6Hz), 7.89 (s, 1H), 7.66 (m, 3H), 7.43 (m, 2H), 7.28 (m, 1H), 7.17 (s, 1H), 6.61 (s, 1H), 5.83 (s, 1H), 5.78 (m, 1H), 1.79 (d, 3H, *J*= 6.4 Hz). MS (ESI): 511.0 [M+H]⁺.

### Step B - 5-[5-(2-Fluoro-4-pyridinyl)-1H-benzimidazol-1-yl]-3-({(1R)-1-[2-(trifluoromethyl)phenyl]ethyl}oxy)-2-thiophenecarboxamide

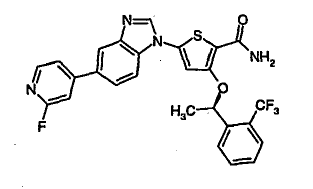

2-Fluoropyridine-4-boronic acid (0.21 g, 1.5 mmol), 5-(5-bromo-1*H* benzimidazol-1-yl)-3-({(1*R*)-1-[2-(trifluoromethyl)phenyl]ethyl}oxy)thiophene-2-carboxamide (0.505 g, 0.99 mmol), and sodium carbonate (1 N in water, 3.4 mL) were combined in *N*,*N*-dimethylacetamide (10 mL). 1,1'-bisdiphenylphosphino-ferrocene dichloropalladium (II) (0.078 g, 0.1 mmol) was added and the reaction mixture was stirred under N₂ while heating at 80 °C for 2 h. The mixture was then cooled and partitioned between EtOAc and water. The aqueous layer was extracted twice with EtOAc. The combined organics were dried over MgSO₄ concentrated onto silica gel and purified by column chromatography (0 to 70% EtOAc:hexanes) to afford 0.34.3 g (67%) of the title compound as a light tan solid. ¹H NMR (400 MHz, CDCl₃): δ 8.29 (d, 1H. *J*= 5.3 Hz), 8.12 (s, 2H), 7.68 (m, 4H), 7.51 (m, 3H), 7.21 (s, 1H), 7.18 (s, 1H), 6.71 (s, 1H), 5.85 (m, 2H), 1.82 (d, 3H, *J*= 5.9 Hz); MS (ESI): 527.2 [M+H]⁺.

### Step C - 5-{5-[2-(4-Methyl-1-piperazinyl)-4-pyridinyl]-1H-benzimidazo/-1-yl}-3-({(1R)-1-[2-(trifluoromethyl)phenyl]ethyl}oxy)-2-thiophenecarboxamide .

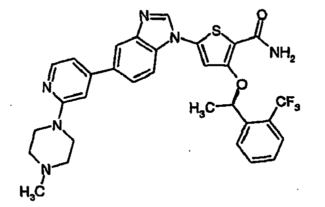

A solution of 5-[5-(2-fluoro-4-pyridinyl)-1H-benzimidazol-1-yl]-3-({(1R)-1-[2-(trifluoromethyl)phenyl]ethyl}oxy)-2-thiophenecarboxamide (0.243 g, 0.44 mmol), 1-methyl piperazine (0.4 mL, 3.6 mmol) and ethanol (95%, 0.4 mL) was heated in a Personal Chemistry microwave at 180 °C for 36 min after which time the mixture was diluted with DCM, washed with water, dried over MgSO₄ and concentrated onto silica gel. Purification by column chromatography (10-100% 90/9/1 DCM/MeOH/ammonium hydroxide:DCM) provided 0.200 g (71%) of the title compound as a tan solid. ¹H NMR (400 MHz, CRCl3): δ 8.26 (d, 1H, *J*= 5.1 Hz), 8.04 (s, 1H), 7.98 (s, 1H), 7.74 (d, 1H, *J*= 7.9 Hz), 7.71 (d, 1H, *J*= 7.9 Hz), 7.64 (t, 1H, *J*= 7.6 Hz), 7.57 (dd, 1H, *J*= 8.5, 1.6 Hz), 7.50 (m, 2H), 7.21 (br s, 1H), 6.92 (d, 1H, *J*= 5.1 Hz), 6.88 (s, 1H), 6.68 (s, 1H). 5.87 (q, 1H, *J*= 6.3 Hz), 5.81 (br s, 1H), 3.74 (m, 4H), 2.67 (m, 4H), 2.39 (s, 3H), 1.82 (d, 3H, *J*= 6.4 Hz); MS (ESI): 607.3 [M+H]⁺.

### Example 3: 5-(5-{2-[(1-Methyl-4-piperidinyl)amino]-4-pyridiny}-1H-benzimidazol-1-yl)-3-({(1R)-1-[2-(trifluoromethyl)phenyl]ethyl}oxy)-2-thiophenecarboxamide

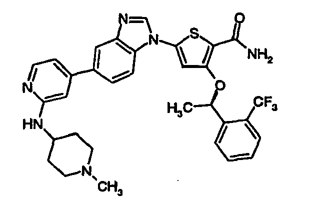

A solution of 5-[5-(2-fluoro-4-pyridinyl)-1H-benzimidazol-1-yl]-3-({(1R)-1-[2-(trifluoromethyl)phenyl]ethyl}oxy)-2-thiophenecarboxamide (0.180 g, 0.34 mmol), 4-amino-1-methylpiperidine (0.4 mL) and ethanol (95%, 0.4 mL) was heated in a Personal Chemistry microwave at 180 °C for 40 min after which time the mixture was diluted with DCM and washed with water. The aqueous layer was extracted with DCM (x4), the organics were combined, dried over MgSO₄ and concentrated onto silica gel. Purification by column chromatography (10-100% 90/9/1 DCM/MeOH/ammonium hydroxide:DCM) provided 0.120 g (57 %) of the title compound as a light yellow solid. ¹H NMR (400 MHz, CDCl₃): δ 8.13 (d, 1H, *J*= 5.3 Hz), 8.02 (d, 1H, *J*= 1.1 Hz), 7.96 (s, 1H), 7.72 (m, 2H), 7.64 (t, 1H, *J*= 7.6 Hz), 7.56 (dd, 1H, *J*= 8.6, 1.5 Hz), 7.48 (m, 2H), 7.21 (br s, 1H), 6.82 (dd, 1H, *J*= 5.3, 1.3 Hz), 6.67 (s, 1H), 6.59 (s, 1H). 6.09 (br s, 1H), 5.87 (q, 1H. *J*= 6.2 Hz), 4.53 (d, 1H, *J*= 8.1 Hz), 3.73 (m, 1H), 2.84 (d, 2H, *J*= 10.3 Hz), 2.32 (s, 3H), 2.21 (t, 2H, *J*= 10.9 Hz), 2.12 (d, 2H, *J*= 13.0 Hz), 1.81 (d, 3H, *J*= 6.2 Hz), 1.60 (m, 2H); MS (ESI): 621.1 [M+H]⁺.

### Example 4: 5-[5-(2-Amino-4-pyridinyl)-1H-benzimidazol-1-yl]-3-({(1R)-1-[2-(trifluoromethyl)phenyl]ethyl}oxy)-2-thiophenecarboxamide

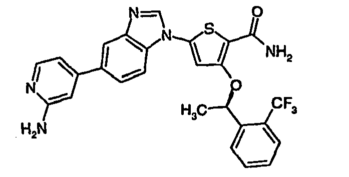

### Step A - Methyl 5-[5-(2-chloro-4-pyridinyl)-1H-benzimidazol-1-yl]-3({(1R)-1-{2-(trifluoromethy/)phenyl]ethyl}oxy)-2-thiophenecarboxy/ate

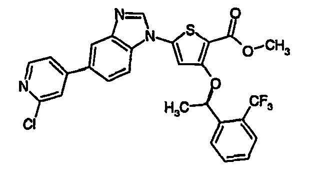

2-Chloro-4-(4,4,5,5-tetramethyl-1,3,2-dioxaborolan-2-yl)-pyridine (0.501 g, 2.09 mmol), methyl 5-(5-bromo-1*H*-benzimidazol-1-yl)-3-({(1*R*)-1-[2-(trifluoromethyl)phenyl]ethyl}oxy)thiophene-2-carboxytate (1.00 g, 1.90 mmol), and sodium carbonate (1 N in water, 7.5 mL) were combined in *N,N-*dimethylacetamide (15 mL). 1,1'- bisdiphenylphosphino-ferrocene dichloropalladium (II) (0.233 g, 0.285 mmol) was added and the reaction mixture was stirred under N₂ while heating at 80 °C for 4 h. The mixture was then cooled and partitioned between 5:1 DCM:MeOH and saturated aqueous NaHCO_{3.} The organic layer was washed with twice with brine, dried over Na₂SO₄, and concentrated onto Celite. Purification by column chromatography (20 to 100% EtOAc in Hexanes with 0.5% triethylamine) provided 0.520 g (49%) of the title compound as an yellow solid. ¹H NMR (400 MHz, CDCl₃): δ 8.43 (d, 1H, *J*= 5.3 Hz), 8.04 (d, 2H, *J*= 13.9 Hz), 7.90 (d, 1H, *J*= 7.9 Hz), 7.66 (d, 1H, *J*= 7.9 Hz), 7.58 (m, 4H), 7.47 (dd, 1H, *J*= 1.1, 5.1 Hz), 7.41 (t, 1H, *J*= 7.6 Hz), 6.78 (s, 1H), 5.81 (q, 1H, *J*= 6.2 Hz), 3.92 (s, 3H), 1.77 (d, 3H, *J*= 6.2 Hz); MS (ESI): 558.1 [M+H]⁺.

### Step B - Methyl 5-(5-{2-[(diphenylmethylidene)amino]-4-pyridinyl}-1H-benzimidazol-1-yl)-3({(1R)-1-[2-(trifluoromethyl)phenyl]ethyl)oxy)-2-thiophenecarboxylate

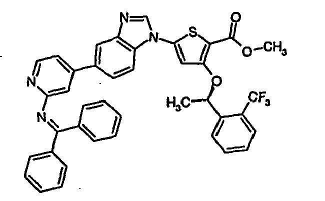

To a degassed solution of methyl 5-[5-(2-chtoro-4-pyridinyl)-1H-benzimidazol-1-yl]-3-({(1R)-1-[2-(triftuoromethyl)phenyl]ethyl}oxy)-2-thiophenecarboxylate (0.250 g, 0.45 mmol) and benzophenone imine (0.09 mL, 0.54 mmol) in 1,4-dioxane (2 mL), cesium carbonate (367 mg, 1.13 mmol) was added followed by 4,5-bis(diphenylphosphino)-9,9-dimethyl-xanthene (10.0 mg, 0.018 mmol) and tris(dibenzylidineacetone)dipalladium (0) (8.0 mg, 0.009 mmol). The reaction mixture was heated to 90 °C for 24 h. The reaction mixture was then diluted with EtOAc, concentrated onto silica gel and purified by column chromatography (0 to 60% EtOAc:hexanes) to afford 0.255 g (83%) of the title compound as an off white solid. MS (ESI): 703.3 [M+H]⁺.

### Step C - Methyl 5-[5-(2-amino-4-pyridinyl)-1H-benzimidazol-1-yl]-3-({(1R)-1-[2-(trifluoromethyl)phenyl]ethyl}oxy)-2-thiophenecarboxylate

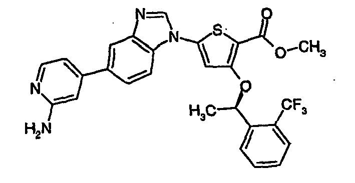

To a solution of methyl 5-(5-{2-[(diphenylmethylidene)amino]-4-pyridinyl}-1H-benzimidazol-1-yl)-3-({(1R)-1-[2-(trifluoromethyl)phenyl]ethyl}oxy)-2-thiophenecarboxylate (0.160 mg, 0.228 mmol) in tetrahydrofuran (6 mL), HCl (2N, aqueous, 3.0 mL) was added and the solution was stirred at room temperature overnight. The reaction mixture was then diluted with DCM, washed with saturated aqueous NaHCO₃ and dried over MgSO₄. The filtrate was evaporated onto silica gel. Purification by column chromatography (10 to 100% 1/9/90 ammonium hydroxide/MeOH/DCM: DCM) provided 0.107 g (88 %) of the title compound as an off-white solid. MS (ESI): 539.2 [M+H]⁺.

### Step D - 5-[5-(2-Amino-4-pyridinyl}-1H-benzimidazo/-1-yl]-3-({(1R)-1-[2-(trifluoromethyl}phenyl]ethyl}oxy)-2-thiophenecarboxamide

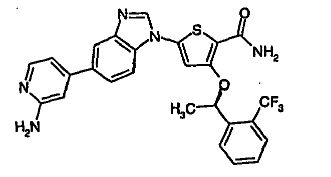

The title compound was prepared from methyl 5-[5-(2-amino-4-pyridinyl)-1H-benzimidazol-1-yl]-3-({(1R)-1-[2-(trifluoromethyl)phenyl]ethyl}oxy)-2-thiophenecarboxylate (0.107 g, 0.199 mmol) and 8 mL 7 N ammonia in MeOH heating in a sealed vessel at 85 °C for 10 h. The mixture was evaporated onto silica gel and purified by column chromatography (10 to 90% 1/9/90 ammonium hydroxide/MeOH/DCM: DCM) to give 0.085 g (82 %) of the desired product as a white solid. ¹H NMR (400 MHz, DMSO-d₆) : δ 8.58 (s, 1H), 7.98 (s, 1H), 7.92 (m, 2H), 7.83 (br s, 1H), 7.76 (m, 2H), 7.57 (m, 3H), 7.14 (s, 1H), 7.12 (br s, 1H), 6.84 (d, 1H, *J*= 5.3), 6.75 (s, 1H), 5.95 (m, 3H), 1.73 (d, 3H, *J*= 6.0 Hz). HRMS C₂₆H₂₁N₅O₂F₃S: [M+H]⁺ calc'd. 524.1368, found 524.1367.

### Example 5: 5-[5-(1H-Pyrazol-4-yl)-1H-Abenzimidazol-1-yl]-3-({(1R)-1-[2-(trifluoromethyl)phenyl]ethyl}oxy)-2-thiophenecarboxamide

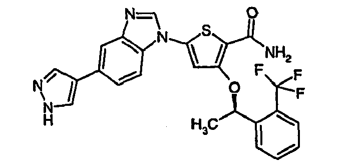

### Step A - Methy/ 5-[5-(1H-pyrazo/-4-yl}-1H-benzimidazo/-1-yl]-3-({(1R)-1-[2-(trifluoromethyl)phenyl]ethyl}oxy)-2-thiophenecarboxylate

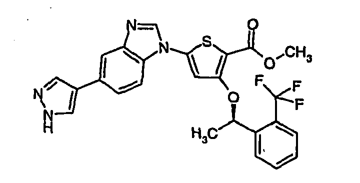

4-(4,4,5,5-Tetramethyl-1,3,2-dioxaborolan-2-yl)-1*H*-pyrazole (148 mg, 0.76 mmol) in *N,N*-dimethylacetamide (3 mL) was treated with aqueous 1 N sodium carbonate (1.5 mL, 1.5 mmol), Methyl 5-(5-bromo-1H-benzimidazol-1-yl)-3-({(1R)-1-[2-(trifluoromethyl)-phenyl]ethyl}oxy)thiophene-2-carboxylate (200 mg, 0.38 mmol) and 1,1'-bis diphenylphosphinoferrocene dichloropalladium(II) DCM adduct (70 mg, 0.07 mmol) and heated to 80 °C for 2 h. After cooling to room temperature the reaction mixture was partitioned between 5:1 chloroform:MeOH and saturated aqueous NaHCO₃. The organic layer was washed with brine (x 2). The layers were separated and the organic layer was dried over Na₂SO₄. The solution was filtered and concentrated and placed on a vacuum pump overnight to remove remaining *N,N-*dimethylacetamide. The crude compound was purified by column chromatography (50-100% EtOAc in hexanes, with 0.5% triethylamine) to yield 132 mg of the title compound as a foam. MS (APCI): 512.9 [M+H]⁺.

### Step B - 5-[5-(1H-Pyrazo/-4-yl}-1H-benzimidazol-1-y/]-3-({(1R)-1-[2-(trifluoromethyl)phenyl]ethyl}loxy)-2-thiophenecarboxamide

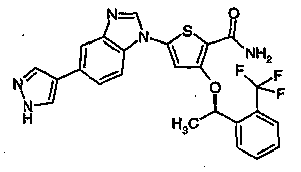

The title compound was prepared from methyl 5-[5-(1H-pyrazol-4-yl)-1H-benzimidazol-1-yl]-3-({(1R)-1-[2-(trifluoromethyl)phenyl]ethyl}oxy)-2-thiophenecarboxylate by a procedure analogous to Example 1, Step E. ¹H NMR (400 MHz, CDCl₃): δ7.91 (m, 4H), 7.72 (d, 1H, *J*= 7.9 Hz), 7.69 (d, 1H, *J*= 8.0 Hz), 7.45 (m, 3H), 7.20 (br s, 1H), 5.91 (br s, 1H), 5.85 (q, 1H, *J*= 6.2 Hz), 1.80 (d, 3H, *J*= 6.0 Hz). HRMS C₂₄H₁₉N₅O₂F₃S: [M+H]⁺ calc'd. 498.1212, found 498.1204.

### Example 6: 3-{[(1R)-1-(2-Chlorophenyl)ethyl]oxy}-5-(5-{6-[(1-methyl-4-piperidinyl)amino]-3-pyridinyl}-1H-benzimidazol-1-yl)-2-thiophenecarboxamide

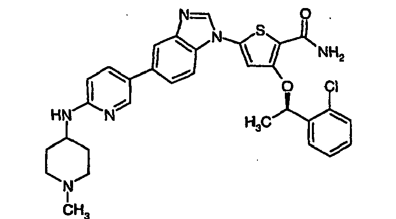

### Step A - Methy/ 5-[(4-bromo-2-nitrophenyl)amino]-3-{[(1R)-1-(2-ch/orophenyl)ethyl]oxy}thiophene-2-carboxy/ate

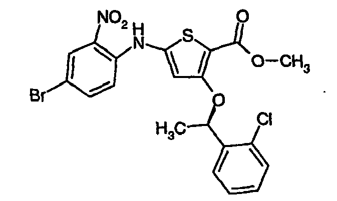

The title compound was prepared from methyl 5-amino-3-{[(1*R*)-1-(2-chlorophenyl)ethyl]oxy}-2-thiophenecarboxylate by a procedure analogous to **Example 1,** Step A. MS (ESI): 534.0 [M+Na]⁺.

### Step B - Methy/ 5-[(2-amino-4-bromophenyl)amino]-3-{{(1R)-1-(2-ch/orophenyl)ethy/]oxy}thiophene-2-carboxy/ate

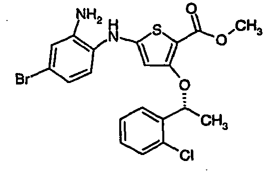

The title compound was prepared from methyl 5-[(4-bromo-2-nitrophenyl)amino]-3-{[(1*R*)-1-(2-chlorophenyl)ethyl]oxy}thiophene-2-carboxylate by a procedure analogous to **Example 1,** Step B. MS (ESI): 482.0 [M+H]⁺.

### Step C - Methy/ 5-(5-bromo-1H-benzimidazo/-1-y/)-3-{[(1R)-1-(2-chlorophenyl)ethyl]oxy}thiophene-2-carboxylate

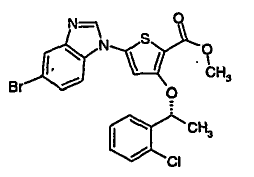

The title compound was prepared from methyl 5-[(2-amino-4-bromophenyl)amino]-3-{[(1*R*)-1-(2-chlorophenyl)ethyl]oxy}thiophene-2-carboxylate by a procedure analogous to **Example 1,** Step C. ¹H NMR (300 MHz, CDCl₃): δ 8.02 (s, 1H), 7.70 (dd, 1H, *J*= 7.6, 1.8 Hz), 7.51-7.28 (m, 6H), 6.74(s, 1H), 5.86 (q, 1H, *J*= 6.3 Hz), 3.97 (s, 3H), 1.79 (d, 3H, *J*= 6.5 Hz); MS (APCI): 492.79 [M+H]⁺.

### Step D - 5-(5-Bromo-1H-benzimidazol-1-yl)-3-{[(1R)-1-(2-chlorophenyl)ethyl]-oxy}thiophene-2-carboxamide

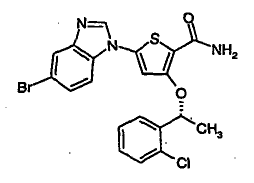

The title compound was prepared from methyl 5-(5-bromo-1*H*-benzimidazol-1-yl)-3-{[(1*R*)-1-(2-chlorophenyl)ethyl]oxy}thiophene-2-carboxylate by a procedure analogous to Example 2, Step A. ¹H NMR (400 MHz, CDCl₃): δ 8.00 (s, 1H), 7.97 (d, *J*=1.3Hz, 1H), 7.46-7.41 (m, 3H), 7.35-7.26 (m, 3H), 7.17 (br s, 1H), 6.60 (s, 1H), 5.85 (q, *J*= 6.4 Hz, 1H), 5.75 (br s, 1H), 1.76 (d, *J*= 6.4 Hz, 3H). MS (APCI): 478.0 [M+H]⁺.

### Step E - 3-{[(1R)-1-(2-Chlorophenyl)ethyl]oxy}-5-[5-(6-fluoropyridin-3-yl)-1H-benzimidazol-1-yl]thiophene-2-carboxamide

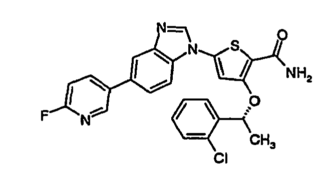

The title compound was prepared from 5-(5-bromo-1*H*-benzimidazol-1-yl)-3-{[(1*R*)-1-(2-chlorophenyl)ethyl]oxy}thiophene-2-carboxamide and 2-fluoropyridine-4-boronic acid by a procedure analogous to **Example 2,** Step B. ¹H NMR (400 MHz, CDCl₃): δ 8.47 (s, 1H), 8.02 (m, 2H), 7.49 (m, 4H), 7.34 (m, 2H), 7.21 (br s, 1H), 7.03 (m, 1H), 6.67 (s, 1H), 5.89 (m, 1H), 5.76 (br s, 1H), 1.79 (d, 3H, *J*= 5.6 Hz); MS (ESI): 493.1 [M+H]⁺.

### Step F- 3-{[(1R)-1-(2-Chlorophenyl)ethyl]oxy}-5-(5-{6-[(1-methyl-4-piperidinyl)amino]-3-pyridinyl}-1H-benzimidazol-1-yl)-2-thiophenecarboxamide

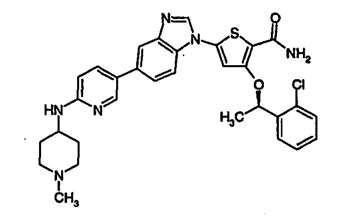

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide and 1-methyl-4-piperidinamine by a procedure analogous to **Example 3.** ¹H NMR (400 MHz, CDCl₃): δ 8.35 (d, 1H, *J*= 2.0 Hz), 7.96 (s, 1H), 7.90 (s, 1H), 7.68-7.66 (m, 1H), 7.50-7.41 (m, 4H), 7.35-7.20 (m, 3H), 6.61 (s, 1H), 6.46 (d, 1H, *J*= 8.4 Hz), 5.88-5.75 (m, 1H), 4.47-4.45 (m, 1H), 3.75 (br s, 1H), 2.96-2.88 (m, 2H), 2.36 (s, 3H), 2.30-2.08 (m, 6H), 1.77 (d, 3H, *J*= 6.4 Hz); MS (ESI): 587 [M+H]⁺.

### Example 7: 3-{[(1R)-1-(2-Chlorophenyl)ethyl]oxy}-5-{5-[6-(1-piperazinyl)-3-pyridinyl]-1H-benzimidazol-1-yl}-2-thiophenecarboxamide

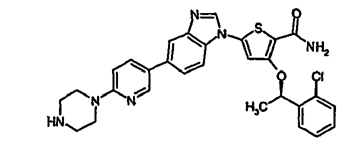

### Step A - 1,1-Dimethy/ethy/ 4-[5-(1-{4-{[(1R)-1-(2-ch/orophenyl)ethyl]oxy}-5-[(methyloxy)carbonyl]-2-thienyl}-1H-benzimidazol-5-yl)-2-pyridinyl]-1-piperazinecarboxylate

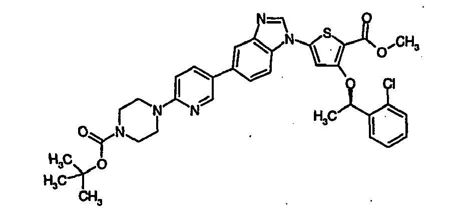

The title compound was prepared from methyl 5-(5-bromo-1H-benzimidazol-1-yl)-3-{[(1R)-1-(2-chlorophenyl)ethyl]oxy}thiophene-2-carboxylate and 1,1-dimethylethyl 4-[5-(4,4,5,5-tetramethyl-1,3,2-dioxaborolan-2-yl)-2-pyridinyl]-1-piperazinecarboxylate by a procedure analogous to **Example 1,** Step D. MS (APCI): 674.1 [M+H]⁺.

### Step B - 1,1-Dimethylethy/ 4-{5-[1-(5-(aminocarbonyl)-4-{[(1R)-1-(2-chlorophenyl)ethyl]oxy}-2-thienyl)-1H-benzimidazol-5-yl]-2-pyridinyl}-1-piperazinecarboxylate

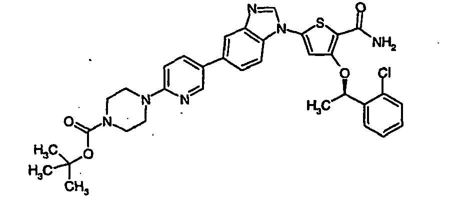

The title compound was prepared from 1,1-dimethylethyl 4-[5-(1-{4-{[(1R)-1-(2-chlorophenyl)ethyl]oxy}-5-[(methyloxy)carbonyl]-2-thienyl}-1H-benzimidazol-5-yl)-2-pyridinyl]-1-piperazinecarboxylate by a procedure analogous to **Example 1,** Step E. MS (APCI): 659.1 [M+H]⁺.

### Step C - 3-{[(1R)-1-(2-Chlorophenyl)ethyl]oxy}-5-{5-[6-(1-piperazinyl)-3-pyridinyl]-1H-benzimidazol-1-yl}-2-thiophenecarboxamide

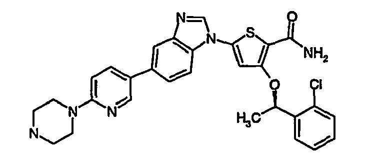

The title compound was prepared from 1,1-dimethylethyl 4-{5-[1-(5-(aminocarbonyl)-4-{[(1R)-1-(2-chlorophenyl)ethyl]oxy}-2-thienyl)-1H-benzimidazol-5-yl]-2-pyridinyl}-1-piperazinecarboxylate by a procedure analogous to **Example 1,** Step F. ¹H NMR (400 MHz, CDCl₃): δ 8.63 (s, 1H), 8.54 (d, 1H, *J*= 2.5 Hz), 8.02 (s, 1H), 7.96 (dd, 1H, *J*= 8.8, 2.7 Hz), 7.85 (br s, 1H), 7.73 (dd, 1H, *J*= 7.6,1.7 Hz), 7.69-7.55 (m, 3H), 7.51-7.37 (m, 2H), 7.26 (s, 1H), 7.16 (br s, 1H), 6.93 (d, 1H, *J*= 8.8 Hz), 6.04 (q, 1H, *J*= 6.3 Hz), 3.53-3.47 (m, 4H), 2.87-2.81 (m, 4H), 1.78 (d, 3H, *J*= 6.3 Hz); HRMS C₂₉H₂₇ClN₆O₂S: [M+H]⁺ calc'd. 559.1680, found 559.1676.

### Example 8: 3-{[(1R)-1-(2-Chlorophenyl)ethyl]oxy}-5-(5-{1-[3-(4-ethyl-1-piperazinyl)propyl]-1H-pyrazol-4-yl}-1H-benzimidazol-1-yl)-2-thiophenecarboxamide

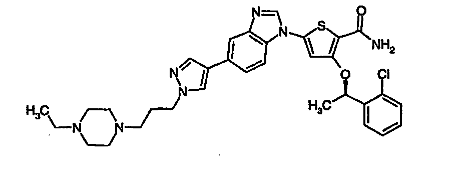

### Step A - Methyl 3-{[(1R)-1-(2-Chloropheny/)ethyl]oxy}-5-[5-(1H-pyrazo/-4-yl)-1H-benzimidazo/-1-yl]-2-thiophenecarboxylate

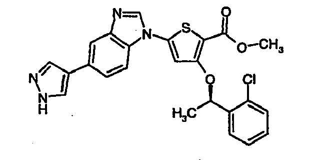

4-(4,4,5,5-Tetramethyl-1,3,2-dioxaborolan-2-yl)-1*H*-pyrazole (355 mg, 1.83 mmol) in *N,N*-dimethylacetamide (15 mL) was treated with aqueous 1 N sodium carbonate (4.5 mL, 4.5 mmol), methyl 5-(5-bromo-1*H*-benzimidazol-1-yl)-3-{[(1*R*)-1-(2-chlorophenyl)ethyl]oxy}thiophene-2-carboxylate (600 mg, 1.22 mmol) and 1,1'-bis diphenylphosphinoferrocene dichloropalladium(II) DCM adduct (149 mg, 0.18 mmol) and heated to 80 °C overnight. After cooling to room temperature the reaction mixture was partitioned between 5:1 chloroform:MeOH and saturated aqueous NaHCO₃. The layers were separated and the organic layer was dried over Na₂SO₄. The solution was filtered and concentrated and placed on a vacuum pump overnight to remove remaining *N,N*-dimethylacetamide. The crude compound was purified by column chromatography (hexanes:EtOAc, with 0.5% triethylamine) to yield 349 mg of the title compound as a foam. MS (APCI): 478.9 [M+H]⁺.

### Step B - Methyl 3-{[(1R)-1-(2-chlorophenyl)ethyl]oxy}-5-(5-{1-[3-(4-ethyl-1-piperazinyl)propyl]-1H-pyrazol-4-yl}-1H-benzimidazol-1-yl)-2-thiophenecarboxylate

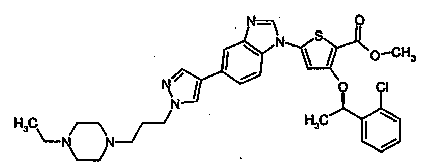

To methyl 3-{[(1*R*)-1-(2-chlorophenyl)ethyl]oxy}-5-[5-(1*H*-pyrazol-4-yl)-1*H-*benzimidazol-1-yl]-2-thiophenecarboxylate (125 mg, 0.26 mmol) in *N,N-*dimethylformamide (2 mL) was added cesium carbonate (136 mg, 0.42 mmol) and stirred for 30 min. Literature-known (Journal of the Chemical Society, Abstracts 1961, 2404-2418) 1-(3-bromopropyl)-4-ethylpiperazine dihydrobromide (165 mg, 0.42 mmol) and more cesium carbonate (136 mg, 0.42 mmol) were added to the reaction and stirred at 70 °C for 1 h. No reaction was noted. More cesium carbonate (170 mg) was added and then the reaction was complete within 1.5 h. The reaction mixture was concentrated onto silica gel and purified by column chromatography (hexane then DCM:90/9/1 DCM/MeOH/ammonium hydroxide) to afford 140 mg of the title compound as a yellow oil. MS (ESI): 633.31 [M+H]⁺.

### Step C - 3-{[(1R)-1-(2-Chlorophenyl)ethyl]oxy}-5-(5-{1-[3-(4-ethyl-1-piperazinyl)propyl]-1H-pyrazo/-4-y/}-1H-benzimidazo/-1-y/)-2-thiophenecarboxamide

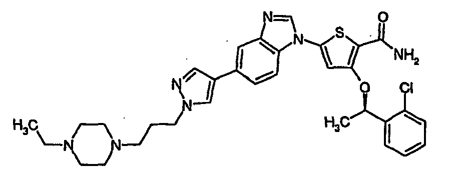

Methyl-3-{[(1*R*)-1-(2-chlorophenyl)ethyl]oxy}-5-(5-{1-[3-(4-ethyl-1-piperazinyl)propyl]-1H-pyrazol-4-yl}-1*H*-benzimidazol-1-yl)-2-thiophenecarboxylate (140 mg, 0.22 mmol) was dissolved in 7 N ammonia in MeOH (6mL) and placed in a sealed vessel. The reaction was heated at 80 °C overnight and cooled to room temperature, at which time the reaction was incomplete. More 7 N ammonia in MeOH (1.5 mL) was added and the reaction was heated at 80 °C for another 4 hours. Silica gel was then added, and the mixture was concentrated and purified by column chromatography (90/9/1 DCM/MeOH/ammonium hydroxide:DCM) to afford 45 mg of the title compound as a beige solid. ¹H NMR (300 MHz, DMSO-d₆): δ 8.58 (s, 1H), 8.28 (s, 1H), 8.01 (s, 1H), 7.98 (s, 1H), 7.80 (br s, 1H), 7.73 (dd, 1H, *J*= 7.5, 1.8 Hz), 7.64 (d, 1H, *J*= 9.7 Hz), 7.56-7.39 (m, 4H), 7.22 (s, 1H), 7.18 (br s, 1H), 6.04 (q, 1H, *J*= 6.3 Hz), 4.17 (t, 2H, *J*= 7.0 Hz), 2.56-2.50 (m, 2H), 2.45-2.25 (m, 10H), 2.06-1.94 (m, 2H), 1.77 (d, 3H, *J*= 6.3 Hz), 1.01 (t, 3H, *J*= 7.1 Hz); HRMS C₃₂H₃₇N₇O₂SCl: [M+H]⁺ calc'd. 618.2412, found 618.2410.

### Example 9:3-{[(1R)-1-(2-Chlorophenyl)ethyl]oxy}-5-[5-(1-methyl-1H-pyrazol-4-yl)-1H-benzimidazol-1-yl]-2-thiophenecarboxamide

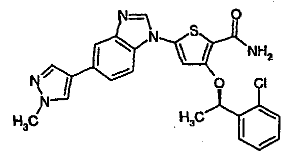

1-Methyl-4(4,4,5,5-tetramethyl-1,3,2-dioxaborolan-2-yl)1H-pyrazole (1.6 g, 7.6 mmol) was suspended in *N,N-*dimethylacetamide (22 mL) and treated with aqueous 1 N sodium carbonate (11.7 mL, 11.7 mmol), 5-(5-bromo-1*H-*benzimidazol-1-yl)-3-{[(1*R*)-1-(2-chlorophenyl)ethyl]oxy}-2-thiophenecarboxamide (2.8 g, 5.9 mmol) and 1,1'-bis diphenylphosphinoferracene dichloropalladium(II) DCM adduct (479 mg, 0.59 mmol) and heated to 80 °C for 30 min. The reaction was allowed to cool to room temperature and partitioned between 5:1 DCM: MeOH and water. The layers were separated and the organic layer was dried over Na₂SO₄. The mixture was filtered, concentrated and placed on vacuum pump at 50 °C overnight to remove residual *N,N*-dimethylacetamide. The oil was dissolved in DCM. Silica gel was added and the mixture was concentrated. The compound was purified by column chromatography (90/9/1 DCM/MeOH/ammonium hydroxide: DCM). The product was triturated in diethyl ether and filtered to yield 2.85g of the title compound as a white solid. Due to an impurity the solid was dissolved in DCM and repurified by column chromatography (EtOAc:MeOH) to yield the product as a white solid (2.76g). ¹H NMR (400 MHz, DMSO-*d₆*): δ 8.54 (s, 1H), 8.19 (s, 1H), 7.95 (s, 1H), 7.93 (s, 1H), 7.81 (br s, 1H), 7.68 (dd, 1H, J = 7.7,1.5 Hz), 7.58 (d, 1H, *J*= 8.5 Hz), 7.52-7.47 (m, 2H), 7.45-7.34 (m, 2H), 7.18 (s, 1H), 7.11 (br s, 1H), 5.99 (q, 1H, *J*= 6.3 Hz), 3.85 (s, 3H), 1.72 (d, 3H, *J*= 6.2 Hz); HRMS C₂₄H₂₀ClN₅O₂S: [M+H]⁺ calc'd. 478.1100, found 478.1102.

### Comparative Examples

Comparative Example numbers 126,127,134 and 156 can be prepared using methods known in the art, including those described in PCT Publication No. WO2004/014899 to SmithKline Beecham.

| **Number** | **Structure** |
|---|---|
| 126 | 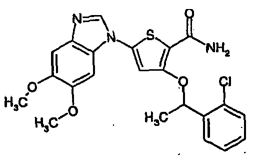 |
| 126 | 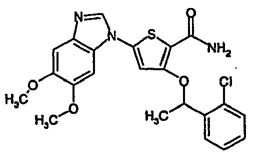 |
| 127 | 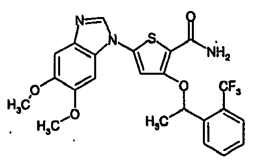 |
| 134 | 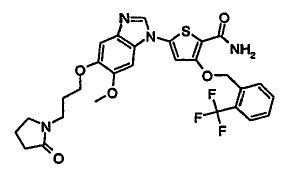 |
| 156 | 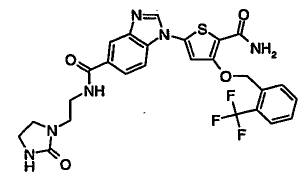 |

### Biological Examples

### I. Assay for inhibition of PLK1

### A. Preparation of 6x N-terminal His-tagged PLK kinase domain

6x N-terminal His-tagged PLK kinase domain (amino acids 21-346 preceded by MKKGHHHHHHD) SEQ ID: No. 1. was prepared from baculovirus infected T. ni cells under polyhedrin promoter control. All procedures were performed at 4 °C. Cells were lysed in 50 mM HEPES, 200 mM NaCl, 50 mM imidazole, 5% glycerol; pH 7.5. The homogenate was centrifuged at 14K rpm in a SLA-1500 rotor for 1 h and the supernatant filtered through a 1.2 micron filter. The supernatant was loaded onto a Nickel chelating Sepharose (Amersham Pharmacia) column and washed with lysis buffer. Protein was eluted using 20%, 30% and 100% buffer B steps where buffer B is 50 mM HEPES, 200 mM NaCl, 300 mM imidazole, 5% glycerol; pH 7.5. Fractions containing PLK were determined by SDS-PAGE. Fractions containing PLK were diluted fivefold with 50 mM HEPES, 1 mM DTT, 5% glycerol; pH 7.5, then loaded on an SP Sepharose (Amersham Pharmacia) column. After washing the column with 50 mM HEPES, 1 mM DTT, 5% glycerol; pH 7.5, PLK was step eluted with 50 mM HEPES, 1 mM DTT, 500 mM NaCl; 5% glycerol; pH 7.5. PLK was concentrated using a 10 kDa molecular weight cutoff membrane and then loaded onto a Superdex 200 gel filtration (Amersham Pharmacia) column equilibrated in 25 mM HEPES, 1 mM DTT, 500 mM NaCl, 5% glycerol; pH 7.5. Fractions containing PLK were determined by SDS-PAGE. PLK was pooled, aliquoted and stored at -80 °C. Samples were quality controlled using mass spectrometry, N-terminal sequencing and amino acid analysis.

### B. Enzyme activity +/- inhibitors was determined as follows:

All measurements were obtained under conditions where signal production increased linearly with time and enzyme. Test compounds were added to white 384-well assay plates (0.1 µL for 10 µL and some 20 µl assays, 1 µL for some 20 µL assays) at variable known concentrations in 100% DMSO. DMSO (1-5% final, as appropriate) and EDTA (65 mM in reaction) were used as controls. Reaction Mix was prepared as follows at 22 °C:
25 mM HEPES, pH 7.2
15 mM MgCl2
1 µM ATP
0.05 µCi/well ³³P-γ ATP (10Ci/mMol)
1 µM substrate peptide (Biotin-Ahx-SFNDTLDFD) SEQ ID:No. 2.
0.15 mg/mLBSA
1 mM DTT
2 nM PLK1 kinase domain (added last)

Reaction Mix (10 or 20 µL) was quickly added to each well immediately following addition of enzyme via automated liquid handlers and incubated 1-1.5 h at 22 °C. The 20 µL enzymatic reactions were stopped with 50 µL of stop mix (50 mM EDTA, 4.0 mg/mLStreptavidin SPA beads in Standard Dulbecco's PBS (without Mg²⁺ and Ca²⁺), 50 µM ATP) per well. The 10 µL reactions were stopped with 10 µL of stop mix (50 mM EDTA, 3.0 mg/mLStreptavidin-coupled SPA Imaging Beads ("LeadSeeker") in Standard Dulbecco's PBS (without Mg²⁺ and Ca²⁺) , 50 µM ATP) per well. Plates were sealed with clear plastic seals, spun at 500 x g for 1 min or settled overnight, and counted in Packard TopCount for 30 seconds/well (regular SPA) or imaged using a Viewlux imager (LeadSeeker SPA). Signal above background (EDTA controls) was converted to percent inhibition relative to that obtained in control (DMSO-only) wells.

### C. Results

The data obtained is reported in **Table 1 and 2** below. In Table 1 and 2, + = pIC₅₀ <6; ++ = pIC₅₀ 6-8; +++ = pIC₅₀ >8.

### II. Methylene Blue Cell Growth Inhibition Assay

Normal human foreskin fibroblasts (HFF), human colon (HCT116, RKO), lung (H460, A549) and breast (MCF7) tumor cell lines were cultured in high glucose DMEM (Life Technologies) containing 10% fetal bovine serum (FBS) at 37 °C in a humidified 5% CO₂, 95% air incubator. Cells were harvested using trypsin/EDTA, counted using a haemocytometer, and plated in 100 µL of culture media per well, at the following densities, in a 96-well tissue culture plate (Falcon 3075): HFF 5,000 cells/well, HCT116 3,000 cells/well, RKO 2,500 cells/well, H460 2,500 cells/well, A549 5,000 cells/well, MCF7 4,000 cells/well. The next day, compounds were diluted in low glucose DMEM containing 100 µg/mL gentamicin, at twice the final required concentration, from 10 mM stock solutions in DMSO. 100 µL/well of these dilutions were added to the 100 µL of media currently in the assay plates. Medium containing 0.6% DMSO was added to control wells. The final concentration of DMSO in all wells was 0.3%. Cells were incubated at 37 °C, 5% CO₂ for 72 h. Medium was removed by aspiration. Cell biomass was estimated by staining cells with 80 µL per well methylene blue (Sigma M9140, 0.5% in 50:50 ethanol:water), and incubation at room temperature for 30-60 min. Stain was removed by aspiration and the plates rinsed by immersion in water, then air-dried. To release stain from the cells, 100 µL of solubilization solution was added (1% N-lauroyl sarcosine, Sodium salt, Sigma L5125, in PBS), and plates were shaken gently for about 30 min. Optical density at 620 nM was measured on a microplate reader. Percent inhibition of cell growth was calculated relative to vehicle treated control wells. Concentration of compound that inhibits 50% of cell growth (IC₅₀) was interpolated using nonlinear regression (Levenberg-Marquardt) and the equation, y = Vₘₐₓ*(1-(x/(K+x))) + Y2, where "K" was equal to the IC₅₀. The data obtained reported in **Table 1** below. In **Table 1,** + = IC₅₀ >1 µM; ++ = IC₅₀ 0.1 -1 µM: +++ = IC₅₀ <0.1 µM.

### III. Cell-Titer Glo Cell Growth Inhibition Assay

A549-L (human lung), BT474 (human breast), Colo 205 (human colon), H1299 (human lung), Hela (human cervix), HCT-116 (human colon), HN5 (human head and neck), HT29 (human colon), LNCap (human prostate), MCF7 (human breast), MDA-MB468 (human breast), MX-1 (human breast), RPMI 8226 (human peripheral blood), SKOV3 (human ovary) and P388 (murine leukemia) cell lines were cultured in RPMI1640 containing 10% fetal bovine serum at 37 °C in a 5% CO₂ incubator. Cell lines were split into T75 flasks (Falcon #353136) two to three days prior to assay set up at density which yields approximately 70-80% confluency at time of harvest for assay. Cells were harvested using 0.25% trypsin with EDTA (Glibco/Invitrogen #25200-056), Cell counts were performed on cell suspensions using Trypan Blue exclusion staining, cells were then plated in 105 uL of culture media per well at the following densities in 96 well Costar #3916 black flat bottom plates: All human cell lines were plated at 500 cells/well. P388 were plated at 250 cells/well. All plates were placed at 5%CO₂ 37 °C overnight and then test compounds were added the following day. One plate of each cell type is treated with CellTiter-Glo (Promega #G7573) for a day 0 proliferation measurement and read as described below. The test compounds are prepared in clear U bottom polypropylene 96 well plates (Falcon #35-1190 with consecutive two fold dilutions. 9 uL of these dilutions was added into each well of the cell plates. The final concentration of DMSO in all wells is 0.15%. Cells were incubated at 37 °C, 5% CO₂ for 72 h. Following 72 h of incubation with compounds each plate was developed and read. CellTiter-Glo reagent was added to assay plates using a volume equivalent to the cell culture volume in the wells. Plates were shaken for approximately two minutes and incubated at room temperature for approximately 15 min and the chemiluminescent signal is read on the Victor V or Envison 2100 reader. Percent inhibition of cell growth was expressed as percent proliferation relative to 100% proliferation (DMSO control). Concentration of compound that inhibited 50% of cell growth (IC₅₀) was analysis by 4 or 6 parameter fit of data using XLfit, value of no cell addition was substracted from all samples for background. The data are shown in Table 2 below. In **Table 2,** + = IC₅₀ >1 µM; ++ = IC₅₀ 0.1 -1 µM : +++ = IC₅₀ <0.1 µM.

### III. Pharmacokinetics (PK)

Preliminary investigation of the intravenous pharmacokinetics of compounds of invention were carried out in fed conscious CD-1 mouse. Compounds were formulated as 1 -10 mg/kg, 5 mL/kg, 1-2 mg/mLsolution **(Table 3).** Twenty one male mice (n= 3 mice per time point) (^{∼}30 g) received dose solution by bolus injection in the tail vein. The formulation for iv were either 20% beta cyclodextrin in saline pH 3.5 - 6 or PEG400 : ethanol : saline (50 : 15 : 35 v/v). Blood samples (^{∼}0.4 mL) were collected terminally by cardiac puncture following anesthetization in a CO₂ chamber at 2, 15, 30, 60 min, 2, 4, and 6 h following iv administration. Blood samples were collected into tubes containing heparin and placed on crushed ice promptly after collection. Plasma was collected by centrifugation, frozen and stored at about -80 °C prior to analysis. Blood or plasma concentrations were determined by LC/MS/MS equipped with an electrospray ion source and using calibration curves for the appropriate matrix. The lower level of quantitation (LLQ) was 10 ng/mL. Blood or plasma concentration-time data were analyzed by non-compartmental methods using the computer program WinNonlin Professional (version 4.1). Area under the blood or plasma concentration-time curve (AUC) was calculated using the linear trapezoidal rule for each incremental trapezoid up to Cmax, and the log trapezoidal rule for each trapezoid thereafter. The terminal elimination rate constant (λz) was derived from the log-linear disposition phase of the concentration-time curve using least-squares regression analysis with visual inspection of the data to determine the appropriate number of terminal data points for regression analysis.

Following iv administration, the total body clearance (CL) was estimated. CL was calculated by dividing the dose by AUC(0-t). The clearance values were assigned as follows. Low <30 mL/min/kg, moderate 30-70 mL/min/kg, high >70 mL/min/kg. The assignment was based on hepatic blood flow i.e. hepatic blood flow of 0-30% low, 30-70% moderate and 70-100% high. The hepatic blood flow in mouse is ^{∼}100 mL/min/kg.

**Table 1.**

| | | MEB | MEB | MEB | MEB | MEB | MEB | MEB |
|---|---|---|---|---|---|---|---|---|
| | PLK1 pIC₅₀ | HCT116 IC₅₀ (µM) | H460 IC₅₀ (µM) | MCF7 IC₅₀ (µM) | A549 IC₅₀ (µM) | RKO IC₅₀ (µM) | MX-1 IC₅₀ (µM) | HFF IC₅₀ (µM) |
| Example 1 | +++ | ++ | ++ | ND | ++ | +++ | ++ | + |
| Example 2 | +++ | ND | ND | ND | ND | ND | ND | ND |
| Example 3 | +++ | ND | ND | ND | ND | ND | ND | ND |
| Example 4 | +++ | +++ | +++ | ND | +++ | +++ | +++ | ++ |
| Example 5 | +++ | +++ | +++ | ND | +++ | +++ | +++ | ++ |
| Example 6 | +++ | ND | ND | ND | ND | ND | ND | ND |
| Example 7 | +++ | ++ | ND | ND | ND | +++ | ND | ND |
| Example 8 | +++ | ND | ND | ND | ND | ND | ND | ND |
| Example 9 | +++ | +++ | +++ | ND | +++ | +++ | +++ | ++ |
| PU4870 Ex126 | +++ | +++ | +++ | + | +++ | +++ | ND | ++ |
| Chiral R of Ex 126 | +++ | +++ | +++ | +++ | +++ | +++ | +++ | ++ |
| Chiral S of Ex 126 | ++ | + | + | + | + | + | ND | + |
| PU4870 Ex 127 | +++ | ND | +++ | ++ | +++ | +++ | ND | + |
| Chiral R of Ex 127 | +++ | +++ | +++ | +++ | +++ | +++ | ND | ND |
| PU4870 Ex 134 | ++ | + | + | + | + | + | ND | + |
| PU4870 Ex 156 | ++ | + | + | + | ND | + | ND | + |

| | | | | | | | | |
|---|---|---|---|---|---|---|---|---|
| ND: Not Determined. | | | | | | | | |

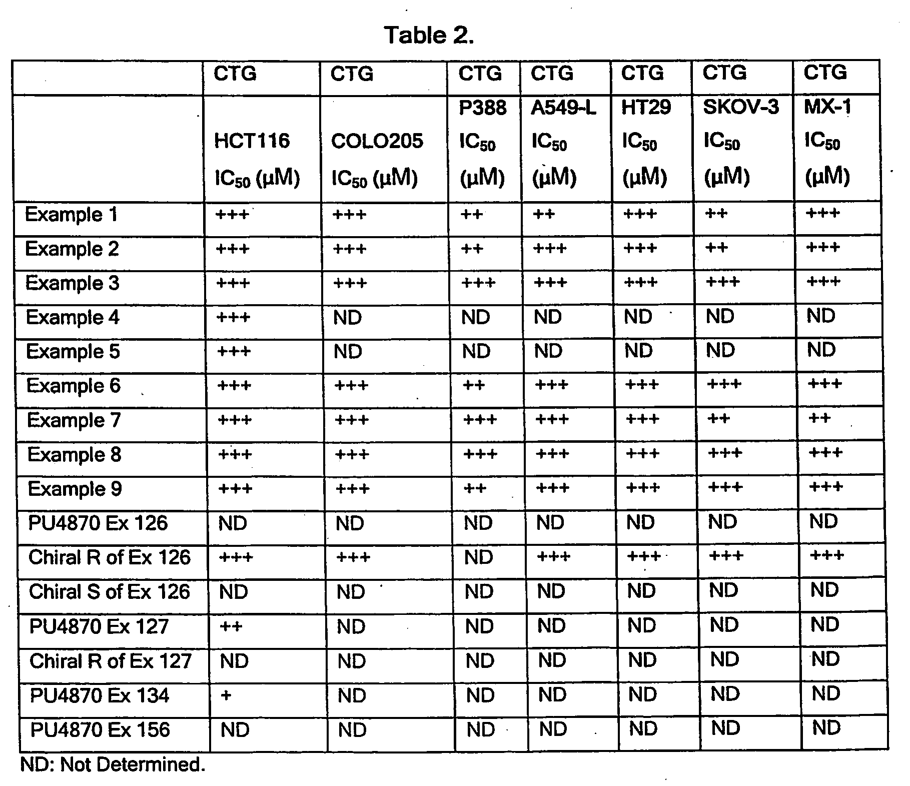

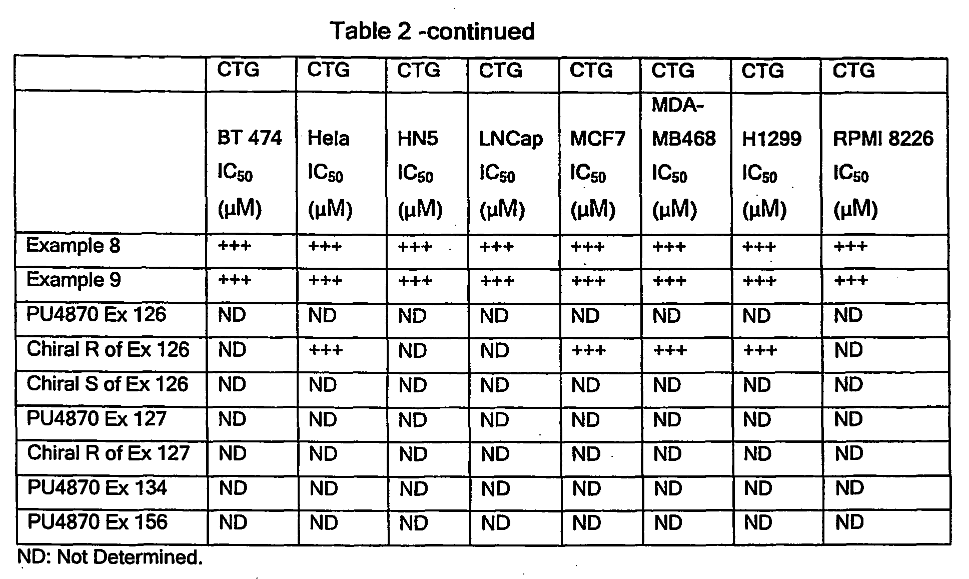

**Table 3.**

| | Formulation | Dose (mg/kg) | Plasma CL (mL/min/kg) | AUC (0-t) (ng*h/mL) |
|---|---|---|---|---|
| Example 1 | 1 MG/ML IN 20% SBE BETA CYCLODEXTRIN, SALINE (PH=4.0) | 4.8 | 23.5 | 3420 |
| Example 2 | 20% SBE BETA CD IN SALINE, PH 5.0 | 4.3 | 32.3 | 2200 |
| Example 3 | 20% SBE BETA CD IN SALINE, PH 3.0-4.0 | 3.9 | 35.6 | 1850 |
| Example 4 | 14%(W/V)SBE CD IN SALINE, 1.4% DMSO AND 22.4% PEG400; PH=4.0 | 2.7 | 37.9 | 1170 |
| Example 5 | 1 MG/ML IN 20% SBE BETA CYCLODEXTRIN, SALINE (PH=4.5-5.0) | 1.9 | 23.1 | 1370 |
| Example 6 | 20% SBE BETA CD IN SALINE, PH 3.5-4.0 | 3.1 | 18.6 | 2780 |
| Example 7 | 20% SBE BETA CD IN SALINE, PH 4.0 | 7.4 | 33 | 3740 |
| Example 8 | 20% SBE BETA CD IN SALINE, PH 4.0 | 3.7 | 43.6 | 1410 |
| Example 9 | 50%PEG400: 15%ETHANOL: 35% SALINE | 4.2 | 12.1 | 5760 |
| PU4870 Ex 126 | | ND | ND | ND |
| Chiral R of Ex 126 | 50% PEG400, 10% 50mM HCL, 15% ETHANOL, 10% SALIN, PH=5-5.5 | 4 | 90.85 | 730* |
| PU4870 Ex 127 | | ND | ND | ND |
| Chiral R of Ex 127 | 1 MG/ML IN 20% SBE BETA CYCLODEXTRIN, SALINE PH=4.5-5 | 2.5 | 98.6 | 400** |

| | | | | |
|---|---|---|---|---|
| ND: Not Determined, * 0-4h, ** 0-1h | | | | |

### Summary of Results

Examples 1-9 showed superior enzyme and/or cell potency over comparative Examples 126 (Chiral S), 134 and 156 in PLK1 enzyme assay and/or methylene blue or cell titer glo cell proliferation assay.

Examples 1-9 showed superior pharmacokinetic (PK) properties in mice over comparative Examples 126 (Chiral R) and 127 (Chiral R) in the test system. When solutions of Examples 1-9 and chiral R of comparative Examples 126 and 127 were dosed in mice via IV administration, Examples 1-9 exhibited lower clearance (CL) and higher area under plasma concentration versus time curve (AUC) when compared to comparative Examples 126 (Chiral R) and 127 (Chiral R).

## Claims

1. A compound selected from: 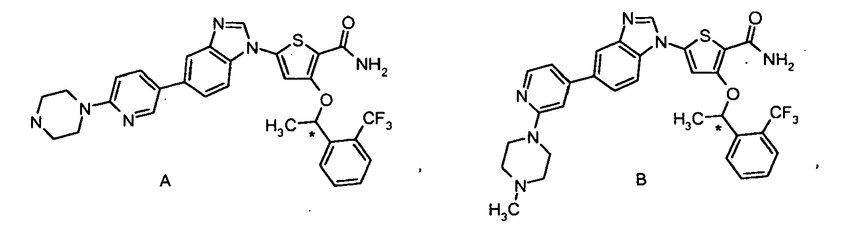 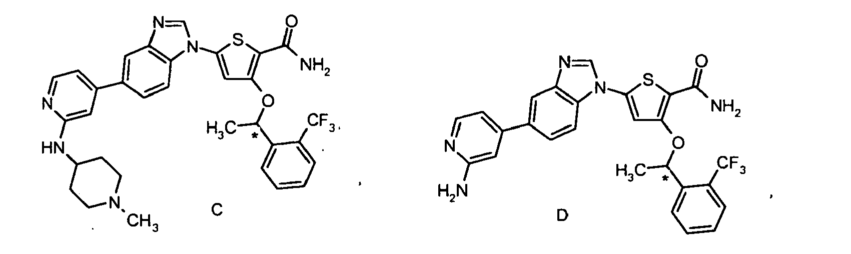 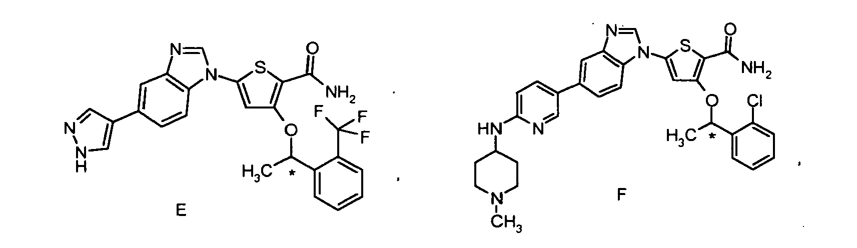 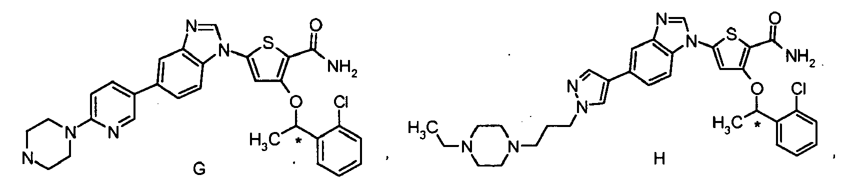 and 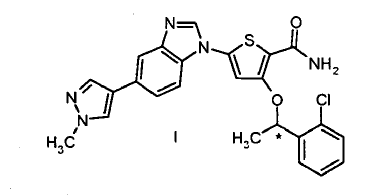 wherein * indicates a chiral carbon;
and pharmaceutically acceptable salts and solvates thereof.

2. A compound according to claim 1, wherein the stereochemistry of the chiral carbon R and pharmaceutically acceptable salts and solvates thereof.

3. An enantiomerically enriched compound having the absolute stereochemistry depicted in A-1: 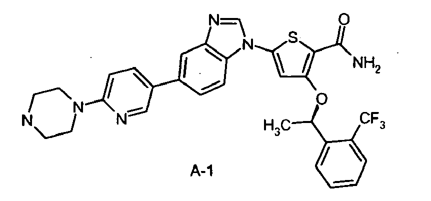 or a pharmaceutically acceptable salt or solvate thereof.

4. An enantiomerically enriched compound having the absolute stereochemistry depicted in B-1: 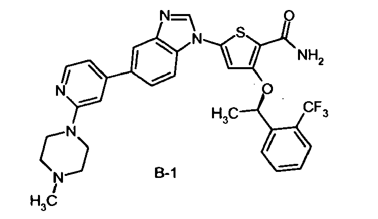 or a pharmaceutically acceptable salt or solvate thereof.

5. An enantiomerically enriched compound having the absolute stereochemistry depicted in C-1: 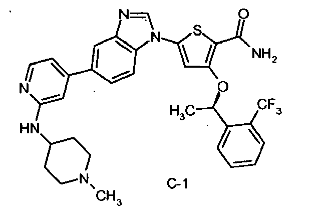 or a pharmaceutically acceptable salt or solvate thereof.

6. An enantiomerically enriched compound having the absolute stereochemistry depicted in D-1: 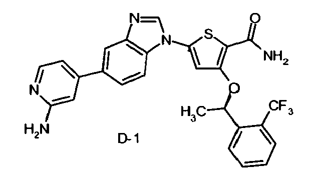 or a pharmaceutically acceptable salt or solvate thereof.

7. An enantiomerically enriched compound having the absolute stereochemistry depicted in E-1: 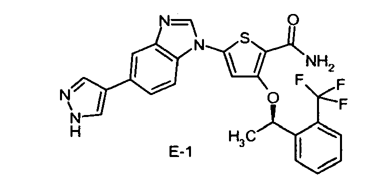 or a pharmaceutically acceptable salt or solvate thereof.

8. An enantiomerically enriched compound having the absolute stereochemistry depicted in F-1: 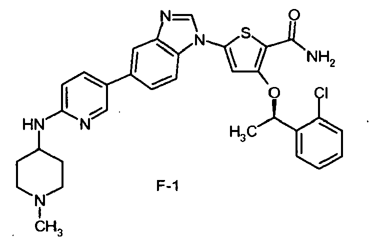 or a pharmaceutically acceptable salt or solvate thereof.

9. An enantiomerically enriched compound having the absolute stereochemistry depicted in G-1: 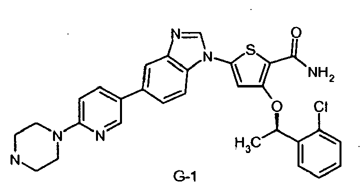 or a pharmaceutically acceptable salt or solvate thereof.

10. An enantiomerically enriched compound having the absolute stereochemistry depicted in H-1: 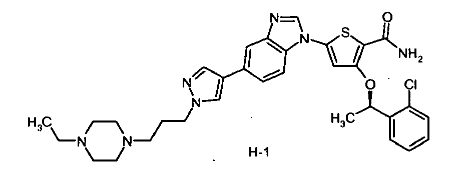 or a pharmaceutically acceptable salt or solvate thereof.

11. An enantiomerically enriched compound having the absolute stereochemistry depicted in I-1:

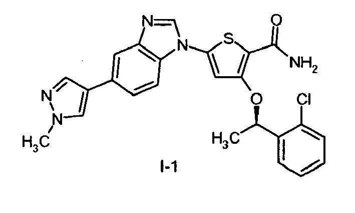

12. A pharmaceutical composition comprising a compound according to any of claims 1-11 or a pharmaceutically acceptable salt or solvate thereof.

13. The pharmaceutical composition according to claim 12 further comprising a pharmaceutically acceptable carrier, diluent or excipient.

14. The pharmaceutical composition according to claim 12 further comprising a chemotherapeutic agent.

15. Use of a compound according to claims 1 - 11 or a pharmaceutically acceptable salt or solvate thereof for the preparation of a medicament for treating a condition mediated by PLK in a mammal in need thereof.

16. Use of a compound according to claims 1 - 11 or a pharmaceutically acceptable salt or solvate thereof for the preparation of a medicament for treating a susceptible neoplasm in a mammal in need thereof.

17. Use according to claim 16, wherein said susceptible neoplasm is selected from the group consisting of breast cancer, colon cancer, lung cancer, prostate cancer, lymphoma, leukemia, endometrial cancer, melanoma, ovarian cancer, pancreatic cancer, squamous carcinoma, carcinoma of the head and neck, and esophageal carcinoma.

18. Use of a compound according to claims 1 - 11 or a pharmaceutically acceptable salt or solvate thereof for the preparation of a medicament for treating breast cancer in a mammal in need thereof.

19. Use of a compound according to claims 1 - 11 or a pharmaceutically acceptable salt or solvate thereof for the preparation of a medicament for treating a condition **characterized by** inappropriate cellular proliferation in a mammal in need thereof.

20. Use of a compound according to claims 1 - 11 or a pharmaceutically acceptable salt or solvate thereof for the preparation of a medicament for inhibiting proliferation of a cell.

21. Use of a compound according to claims 1 - 11 or a pharmaceutically acceptable salt or solvate thereof for the preparation of a medicament for inhibiting mitosis in a cell.

22. The compound according to any of claims 1-11 or a pharmaceutically acceptable salt or solvate thereof for use in therapy.

23. A compound according to any of claims 1-11 or a pharmaceutically acceptable salt or solvate thereof for use in the treatment of a condition mediated by PLK in a mammal in need thereof.

24. A compound according to any of claims 1-11 or a pharmaceutically acceptable salt or solvate thereof for use in the treatment of a susceptible neoplasm in a mammal in need thereof.

25. A compound according to any of claims 1-11 or a pharmaceutically acceptable salt or solvate thereof for use in the treatment of breast cancer in a mammal in need thereof.

26. A compound according to any of claims 1-11 or a pharmaceutically acceptable salt or solvate thereof for use in the treatment of a condition **characterized by** inappropriate cellular proliferation in a mammal in need thereof.

27. A compound according to any of claims 1-11 or a pharmaceutically acceptable salt or solvate thereof for use in inhibiting proliferation of a cell.

28. A compound according to any of claims 1-11 or a pharmaceutically acceptable salt or solvate thereof for use in inhibiting mitosis in a cell.

29. A pharmaceutical composition comprising a compound according to any of claims 1-11 or a pharmaceutically acceptable salt or solvate thereof for use in the treatment of a susceptible neoplasm in a mammal in need thereof.

## Patentansprüche

1. Verbindung ausgewählt aus: 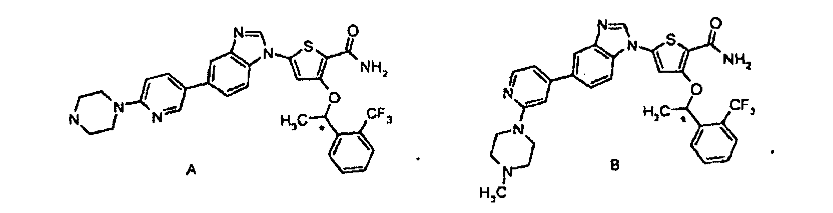 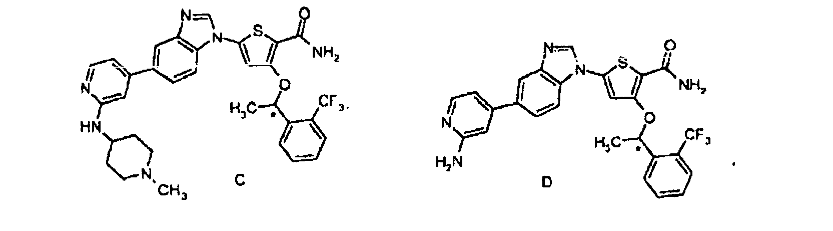 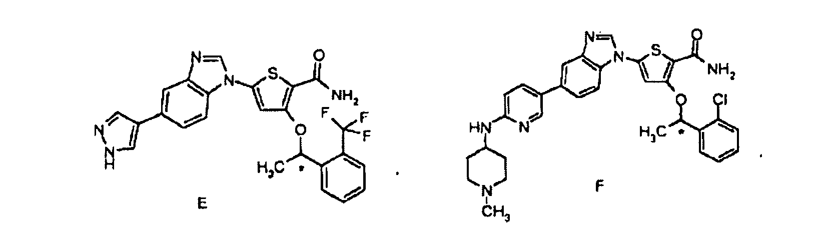 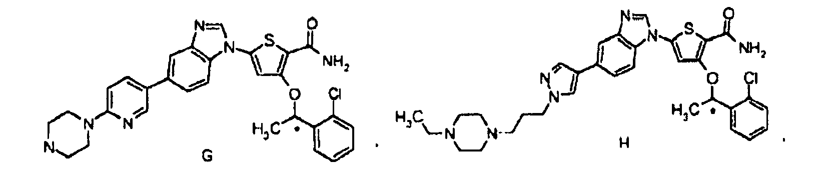 und 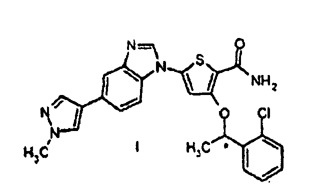 worin * einen chiralen Kohlenstoff kennzeichnet;
und pharmazeutisch annehmbare Salze und Solvate davon.

2. Verbindung gemäß Anspruch 1, worin die Stereochemie des chiralen Kohlenstoffs R ist, und pharmazeutisch annehmbare Salze und Solvate davon.

3. Enantiomerisch angereicherte Verbindung mit der in A-1 dargestellten absoluten Stereochemie: 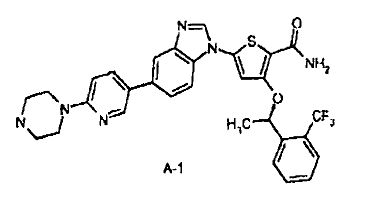 oder ein pharmazeutisch annehmbares Salz oder Solvat davon.

4. Enantiomerisch angereicherte Verbindung mit der in B-1 dargestellten absoluten Stereochemie: 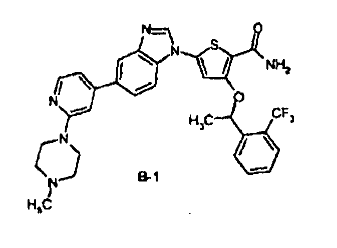 oder ein pharmazeutisch annehmbares Salz oder Solvat davon.

5. Enantiomerisch angereicherte Verbindung mit der in C-1 dargestellten absoluten Stereochemie: 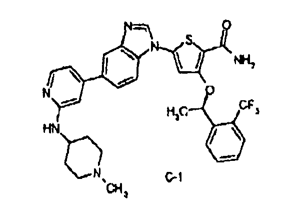 oder ein pharmazeutisch annehmbares Salz oder Solvat davon.

6. Enantiomerisch angereicherte Verbindung mit der in D-1 dargestellten absoluten Stereochemie: 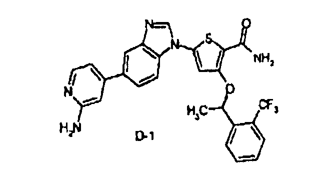 oder ein pharmazeutisch annehmbares Salz oder Solvat davon.

7. Enantiomerisch angereicherte Verbindung mit der in E-1 dargestellten absoluten Stereochemie: 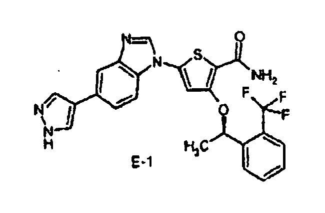 oder ein pharmazeutisch annehmbares Salz oder Solvat davon.

8. Enantiomerisch angereicherte Verbindung mit der in F-1 dargestellten absoluten Stereochemie: 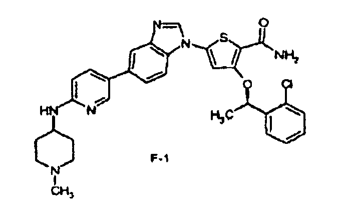 oder ein pharmazeutisch annehmbares Salz oder Solvat davon.

9. Enantiomerisch angereicherte Verbindung mit der in G-1 dargestellten absoluten Stereochemie: 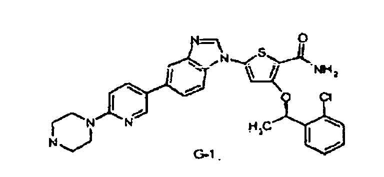 oder ein pharmazeutisch annehmbares Salz oder Solvat davon.

10. Enantiomerisch angereicherte Verbindung mit der in H-1 dargestellten absoluten Stereochemie: 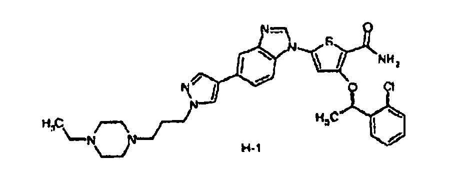 oder ein pharmazeutisch annehmbares Salz oder Solvat davon.

11. Enantiomerisch angereicherte Verbindung mit der in I-1 dargestellten absoluten Stereochemie:

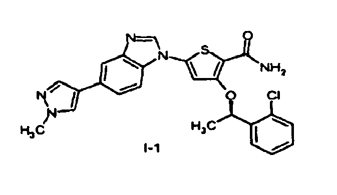

12. Pharmazeutische Zusammensetzung, die eine Verbindung gemäß einem der Ansprüche 1 bis 11 oder ein pharmazeutisch annehmbares Salz oder Solvat davon umfasst.

13. Pharmazeutische Zusammensetzung gemäß Anspruch 12, die ferner einen pharmazeutisch annehmbaren Träger, Verdünnungsstoff oder Exzipienten umfasst.

14. Pharmazeutische Zusammensetzung gemäß Anspruch 12, die ferner ein Chemotherapeutikum umfasst.

15. Verwendung einer Verbindung gemäß den Ansprüchen 1 bis 11 oder eines pharmazeutisch annehmbaren Salzes oder Solvats davon zur Herstellung eines Medikaments zur Behandlung eines Zustands, der durch PLK vermittelt wird, in einem dafür bedürftigen Säuger.

16. Verwendung einer Verbindung gemäß den Ansprüchen 1 bis 11 oder eines pharmazeutisch annehmbaren Salzes oder Solvats davon zur Herstellung eines Medikaments zur Behandlung einer empfindlichen Neoplasie in einem dafür bedürftigen Säuger.

17. Verwendung gemäß Anspruch 16, worin die empfindliche Neoplasie ausgewählt ist aus der Gruppe, bestehend aus Brustkrebs, Darmkrebs, Lungenkrebs, Prostatakrebs, Lymphom, Leukämie, endometrialer Krebs, Melanom, Eierstockkrebs, Bauchspeicheldrüsenkrebs, Plattenepithelkarzinom, Karzinom des Kopfes und Halses und Ösophaguskarzinom.

18. Verwendung einer Verbindung gemäß den Ansprüchen 1 bis 11 oder eines pharmazeutisch annehmbaren Salzes oder Solvats davon zur Herstellung eines Medikaments zur Behandlung von Brustkrebs in einem dafür bedürftigen Säuger.

19. Verwendung einer Verbindung gemäß den Ansprüchen 1 bis 11 oder eines pharmazeutisch annehmbaren Salzes oder Solvats davon zur Herstellung eines Medikaments zur Behandlung eines Zustands, der durch eine unangemessene Zellproliferation **gekennzeichnet** ist, in einem dafür bedürftigen Säuger.

20. Verwendung einer Verbindung gemäß den Ansprüchen 1 bis 11 oder eines pharmazeutisch annehmbaren Salzes oder Solvats davon zur Herstellung eines Medikaments zur Hemmung der Proliferation einer Zelle.

21. Verwendung einer Verbindung gemäß den Ansprüchen 1 bis 11 oder eines pharmazeutisch annehmbaren Salzes oder Solvats davon zur Herstellung eines Medikaments zur Hemmung der Mitose in einer Zelle.

22. Verwendung einer Verbindung gemäß einem der Ansprüche 1 bis 11 oder eines pharmazeutisch annehmbaren Salzes oder Solvats davon zur Verwendung in der Therapie.

23. Verbindung gemäß einem der Ansprüche 1 bis 11 oder ein pharmazeutisch annehmbares Salz oder Solvat davon zur Verwendung in der Behandlung eines Zustands, der durch PLK vermittelt wird, in einem dafür bedürftigen Säuger.

24. Verbindung gemäß einem der Ansprüche 1 bis 11 oder ein pharmazeutisch annehmbares Salz oder Solvat davon zur Verwendung in der Behandlung einer empfindlichen Neoplasie in einem dafür bedürftigen Säuger.

25. Verbindung gemäß einem der Ansprüche 1 bis 11 oder ein pharmazeutisch annehmbares Salz oder Solvat davon zur Verwendung in der Behandlung von Brustkrebs in einem dafür bedürftigen Säuger.

26. Verbindung gemäß einem der Ansprüche 1 bis 11 oder ein pharmazeutisch annehmbares Salz oder Solvat davon zur Verwendung in der Behandlung eines Zustands, der durch unangemessene Zellproliferation **gekennzeichnet** ist, in einem dafür bedürftigen Säuger.

27. Verbindung gemäß einem der Ansprüche 1 bis 11 oder ein pharmazeutisch annehmbares Salz oder Solvat davon zur Verwendung in der Hemmung der Proliferation einer Zelle.

28. Verbindung gemäß einem der Ansprüche 1 bis 11 oder ein pharmazeutisch annehmbares Salz oder Solvat davon zur Verwendung in der Hemmung der Mitose in einer Zelle.

29. Pharmazeutische Zusammensetzung, die eine Verbindung gemäß einem der Ansprüche 1 bis 11 oder ein pharmazeutisch annehmbares Salz oder Solvat davon umfasst, zur Verwendung in der Behandlung einer empfindlichen Neoplasie in einem dafür bedürftigen Säuger.

## Revendications

1. Composé choisi parmi : 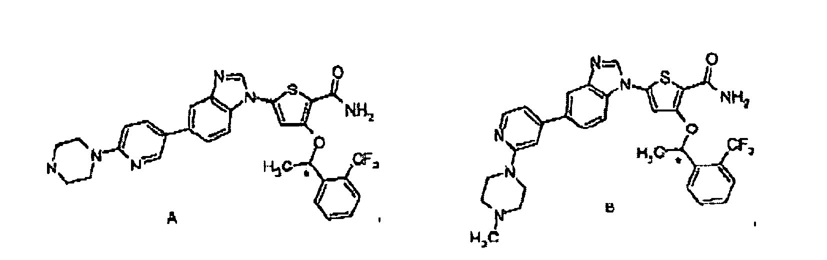 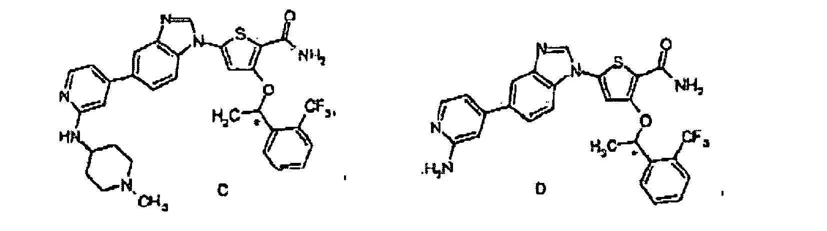 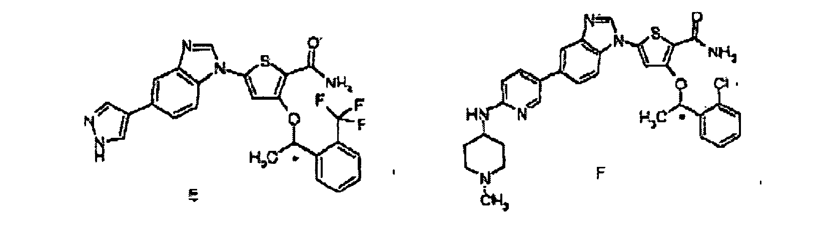 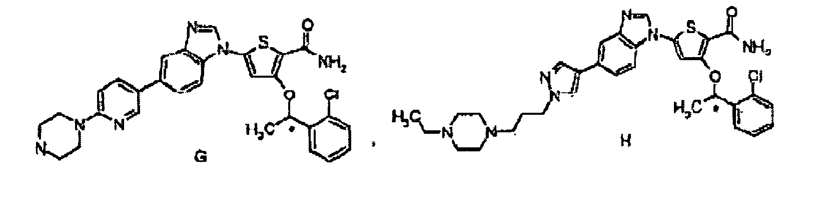 et 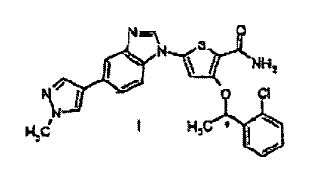 dans lequel * représente un atome de carbone chiral ;
et ses sels et solvates pharmaceutiquement acceptables.

2. Composé selon la revendication 1, dans lequel la stéréochimie de l'atome de carbone chiral est R et ses sels et solvates pharmaceutiquement acceptables.

3. Composé enrichi en énantiomère ayant la stéréochimie absolue représentée par A-1 : 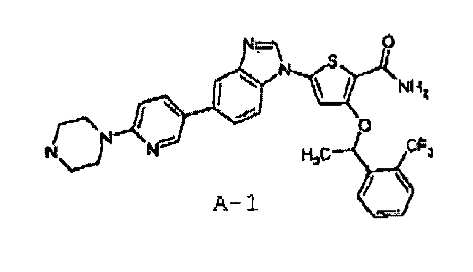 ou son sel ou solvate pharmaceutiquement acceptable.

4. Composé enrichi en énantiomère ayant la stéréochimie absolue représentée par B-1 : 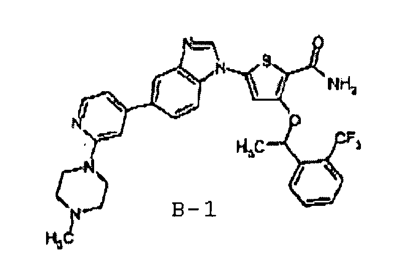 ou son sel ou solvate pharmaceutiquement acceptable.

5. Composé enrichi en énantiomère ayant la stéréochimie absolue représentée par C-1 : 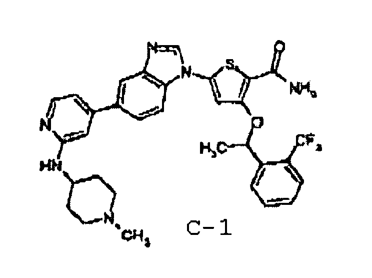 ou son sel ou solvate pharmaceutiquement acceptable.

6. Composé enrichi en énantiomère ayant la stéréochimie absolue représentée par D-1 : 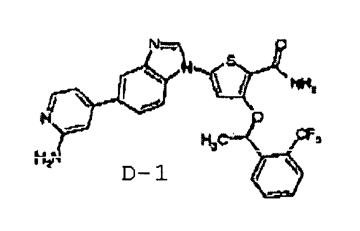 ou son sel ou solvate pharmaceutiquement acceptable.

7. Composé enrichi en énantiomère ayant la stéréochimie absolue représentée par E-1 : 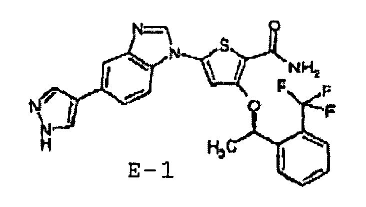 ou son sel ou solvate pharmaceutiquement acceptable.

8. Composé enrichi en énantiomère ayant la stéréochimie absolue représentée par F-1 : 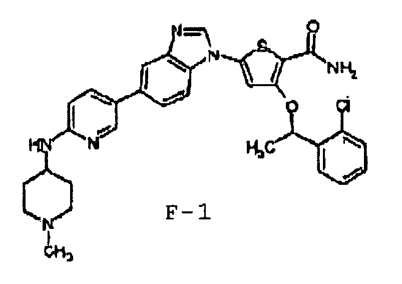 ou son sel ou solvate pharmaceutiquement acceptable.

9. Composé enrichi en énantiomère ayant la stéréochimie absolue représentée par G-1 : 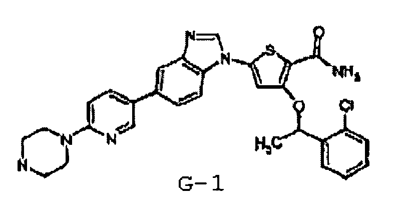 ou son sel ou solvate pharmaceutiquement acceptable.

10. Composé enrichi en énantiomère ayant la stéréochimie absolue représentée par H-1 : 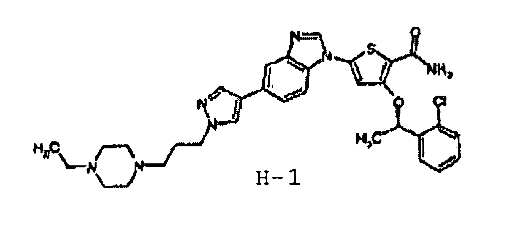 ou son sel ou solvate pharmaceutiquement acceptable.

11. Composé enrichi en énantiomère ayant la stéréochimie absolue représentée par I-1 :

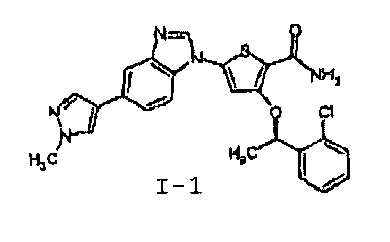

12. Composition pharmaceutique comprenant un composé selon l'une quelconque des revendications 1 à 11 ou son sel ou solvate pharmaceutiquement acceptable.

13. Composition pharmaceutique selon la revendication 12, comprenant en outre un support, un diluant ou un excipient pharmaceutiquement acceptable.

14. Composition pharmaceutique selon la revendication 12, comprenant en outre un agent chimiothérapeutique.

15. Utilisation d'un composé selon les revendications 1 à 11 ou de son sel ou solvate pharmaceutiquement acceptable pour la préparation d'un médicament destiné au traitement d'un trouble induit par la PLK chez un mammifère ayant besoin d'un tel traitement.

16. Utilisation d'un composé selon les revendications 1 à 11 ou de son sel ou solvate pharmaceutiquement acceptable pour la préparation d'un médicament destiné au traitement d'un néoplasme sensible chez un mammifère ayant besoin d'un tel traitement.

17. Utilisation selon la revendication 16, dans laquelle ledit néoplasme sensible est choisi dans le groupe constitué par le cancer du sein, le cancer du côlon, le cancer du poumon, le cancer de la prostate, le lymphome, la leucémie, le cancer de l'endomètre, le mélanome, le cancer de l'ovaire, le cancer du pancréas, le carcinome squameux, le carcinome de la tête et du cou, et le carcinome de l'oesophage.

18. Utilisation d'un composé selon les revendications 1 à 11 ou de son sel ou solvate pharmaceutiquement acceptable pour la préparation d'un médicament destiné au traitement du cancer du sein chez un mammifère ayant besoin d'un tel traitement.

19. Utilisation d'un composé selon les revendications 1 à 11 ou de son sel ou solvate pharmaceutiquement acceptable pour la préparation d'un médicament destiné au traitement d'un trouble **caractérisé par** une prolifération cellulaire inappropriée chez un mammifère ayant besoin d'un tel traitement.

20. Utilisation d'un composé selon les revendications 1 à 11 ou de son sel ou solvate pharmaceutiquement acceptable pour la préparation d'un médicament destiné à inhiber la prolifération d'une cellule.

21. Utilisation d'un composé selon les revendications 1 à 11 ou de son sel ou solvate pharmaceutiquement acceptable pour la préparation d'un médicament destiné à inhiber la mitose dans une cellule.

22. Composé selon l'une quelconque des revendications 1 à 11 ou son sel ou solvate pharmaceutiquement acceptable destiné à un usage thérapeutique.

23. Composé selon l'une quelconque des revendications 1 à 11 ou son sel ou solvate pharmaceutiquement acceptable destiné à être utilisé dans le traitement d'un trouble induit par la PLK chez un mammifère ayant besoin d'un tel traitement.

24. Composé selon l'une quelconque des revendications 1 à 11 ou son sel ou solvate pharmaceutiquement acceptable destiné à être utilisé dans le traitement d'un néoplasme sensible chez un mammifère ayant besoin d'un tel traitement.

25. Composé selon l'une quelconque des revendications 1 à 11 ou son sel ou solvate pharmaceutiquement acceptable destiné à être utilisé dans le traitement du cancer du sein chez un mammifère ayant besoin d'un tel traitement.

26. Composé selon l'une quelconque des revendications 1 à 11 ou son sel ou solvate pharmaceutiquement acceptable destiné à être utilisé dans le traitement d'un trouble **caractérisé par** une prolifération cellulaire inappropriée chez un mammifère ayant besoin d'un tel traitement.

27. Composé selon l'une quelconque des revendications 1 à 11 ou son sel ou solvate pharmaceutiquement acceptable destiné à inhiber la prolifération d'une cellule.

28. Composé selon l'une quelconque des revendications 1 à 11 ou son sel ou solvate pharmaceutiquement acceptable destiné à inhiber la mitose dans une cellule.

29. Composition pharmaceutique comprenant un composé selon l'une quelconque des revendications 1 à 11 ou son sel ou solvate pharmaceutiquement acceptable, destinée à être utilisée dans le traitement d'un néoplasme sensible chez un mammifère ayant besoin d'un tel traitement.
